# EUROPEAN PATENT APPLICATION

(11) **EP 4 582 551 A1**
(43) Date of publication of application: **09.07.2025**
(21) Application number: 23860482.1
(22) Date of filing: 31.08.2023
(51) Int. Cl.: C12Q 1/34, C11D 3/386, C12N 9/20, C12N 15/10, C12N 15/55, C12Q 1/68

(54) **SEARCH METHOD FOR LIPASE**

(30) Priority: 01.09.2022 JP 2022139356
(71) Applicant: Kao Corporation, Chuo-ku, Tokyo 103-8210 (JP)
(72) Inventor: TERAI, Mika, Wakayama-shi, Wakayama 640-8580 (JP); HIOKI, Takahiro, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2023/031814
(87) International publication number: WO 2024/048720

(57) **Abstract**

Provided is a lipase search method which can efficiently search for lipases having resistance to activity inhibition by a surfactant and/or showing excellent detergency in the presence of a surfactant. A lipase search method comprising steps of: designing lipase ancestral sequences by ancestral sequence reconstruction using a lipase sequence group; measuring at least one of a lipase activity of lipases consisting of the designed ancestral sequences in the presence of a surfactant and a detergency thereof in the presence of a surfactant; and comparing measured results with at least one of a lipase activity of a standard lipase in the presence of a surfactant and a detergency thereof in the presence of a surfactant.

## Description

### Field of the Invention

The present invention relates to a lipase search method.

### Background of the Invention

Lipases are useful for various applications, such as laundry cleansing agents, dishwashing cleansing agents, fat/oil fat processing, pulp processing, feed, and pharmaceutical intermediate synthesis. In cleansing, lipases hydrolyze triglycerides to produce fatty acids, thereby contributing to the removal of stains including oil.

Current cleansing compositions and cleansing environments contain various components which inhibit the activity and cleansing effect of lipases, and lipases which function under such conditions are desired. As a lipase useful for cleansing, *Thermomyces lanuginosus-*derived lipase (hereinafter, TLL) is sold under the product name LIPOLASE (registered trademark). Patent Literature 1 discloses that Cedecea sp-16640 strain-derived lipase Lipr139 has superior cleansing performance in comparison with TLL. Patent Literature 2 discloses a mutant of *Proteus* bacterium-derived lipase (hereinafter, PvLip), which has improved cleansing performance in comparison with one or more reference lipolytic enzymes. Patent Literature 3 discloses that metagenome-derived lipase Lipr138 has superior cleansing performance in comparison with TLL. Lipr139, PvLip, and Lipr138 are lipases belonging to the same clade (*Proteus*/*Yersinia* clade) including lipases derived from *Proteus* or *Yersinia* bacteria.

Bacterial lipases are classified into eight families (Non-Patent Literature 1). Of these, many gram-negative bacterium-derived lipases belonging to subfamily I.1 or I.2 require lipase-specific chaperones for folding into active forms (Non-Patent Literature 2), and have an issue in achieving low-cost production by heterologous expression. It has been reported that co-expression with specific chaperones improves heterologous expression (Non-Patent Literature 3); however, the productivity thereof is low, and there is still a major issue in achieving low-cost production by heterologous expression. In contrast, among lipases of subfamily I.1, some of the bacterial lipases belonging to the *Proteus*/*Yersinia* clade do not require specific chaperones, and have advantages in low-cost production by heterologous expression (Non-Patent Literatures 4 and 5). As described above, searching for lipases which allow low-cost production by heterologous expression and exhibit their functions in various cleansing environments is being actively carried out.

(Patent Literature 1) JP-A-2015-523078
(Patent Literature 2) WO 2020/046613
(Patent Literature 3) JP-A-2015-525248
(Non Patent Literature 1) Arpigny JL, Jaeger KE. The Biochemical journal 343 Pt 1(Pt 1) (1999): 177-183.
(Non Patent Literature 2) Hobson, Audrey H., et al. Proceedings of the National Academy of Sciences 90.12 (1993): 5682-5686.
(Non Patent Literature 3) Quyen, ThiDinh, ChiHai Vu, and GiangThi Thu Le. Microbial cell factories 11.1 (2012): 1-12.
(Non Patent Literature 4) Lee, Hong-Weon, et al. Biotechnology letters 22.19 (2000): 1543-1547.
(Non Patent Literature 5) Glogauer, Arnaldo, et al. Microbial cell factories 10.1 (2011): 1-15.

### Summary of the Invention

The present invention provides a lipase search method comprising steps of: designing lipase ancestral sequences by ancestral sequence reconstruction using a lipase sequence group; measuring at least one of a lipase activity of lipases consisting of the designed ancestral sequences in the presence of a surfactant and a detergency thereof in the presence of a surfactant; and comparing measured results with at least one of a lipase activity of a standard lipase in the presence of a surfactant and a detergency thereof in the presence of a surfactant.

### Brief Description of Drawings

Fig. 1 illustrates the HMM profiles of the QGLP clade.
Fig. 2 illustrates the HMM profiles of the *Proteus*/*Yersinia* clade.
Fig. 3 illustrates the lipase activity of lipases for cleansing in an SDS solution.
Fig. 4 illustrates the lipase activity of lipases for cleansing in a Triton X-100 solution.
Fig. 5 illustrates the lipase activity of lipases for cleansing in a model cleansing liquid.
Fig. 6 illustrates the detergency of lipases for cleansing in a model cleansing liquid.
Fig. 7 illustrates the phylogenetic tree of lipase sequence groups closely related to the *Proteus*/*Yersinia* clade.
Fig. 8 illustrates the lipase activity of Lipr139 and lipases of Groups 1, 2, 3, and 4 in an SDS solution.
Fig. 9 illustrates the lipase activity of Lipr139 and lipases of Groups 1, 2, 3, and 4 in a Triton X-100 solution.
Fig. 10 illustrates the lipase activity of Lipr139 and lipases of Groups 1, 2, 3, and 4 in a model cleansing liquid.
Fig. 11 illustrates the detergency of Lipr139 and lipases of Groups 1, 2, 3, and 4 in a model cleansing liquid.
Fig. 12 illustrates the phylogenetic tree of lipase sequence groups closely related to the QGLP clade.
Fig. 13 illustrates the lipase activity of Lipr139 and lipases of Groups 5 and 6 in an SDS solution.
Fig. 14 illustrates the lipase activity of Lipr139 and lipases of Groups 5 and 6 in a Triton X-100 solution.
Fig. 15 illustrates the lipase activity of Lipr139 and lipases of Groups 5 and 6 in a model cleansing liquid.
Fig. 16 illustrates the detergency of Lipr139 and lipases of Groups 5 and 6 in a model cleansing liquid.

### Detailed Description of the Invention

All of the patent literatures, non-patent literatures, and other publications cited in the present specification are incorporated herein by reference in their entirety.

In the present specification, "sequence group" refers to a population of sequences containing at least two amino acid sequences, and the identity between sequences with the lowest identity is 98% or less, preferably 95% or less, more preferably 90% or less, and further more preferably 80% or less.

In the present specification, "lipase" refers to triacylglycerol lipase (EC3.1.1.3), and means an enzyme group having activity to hydrolyze triglycerides to produce fatty acids. The lipase activity can be determined by measuring the rate of increase in absorbance associated with the release of 4-nitrophenol by the hydrolysis of 4-nitrophenyl octanoate. The specific procedure of measuring the lipase activity is described in detail in Reference Example 1 provided later.

In the present specification, "existing lipases" refer to lipases possessed by existing organisms. Hereinafter, the amino acid sequences of the existing lipases are also referred to as the lipase existing sequences. The lipase existing sequences can be obtained from a public sequence database or by screening from nature.

In the present specification, "ancestral lipases" refer to lipases consisting of amino acid sequences estimated to be possessed by a common ancestor derived from the existing sequence of the lipase of each organism derived from the common ancestor based on a rooted phylogenetic tree which shows the protein evolution. Hereinafter, the amino acid sequences of the ancestral lipases are also referred to as the lipase ancestral sequences. The lipase ancestral sequences can be determined by ancestral sequence reconstruction (ASR) based on the existing lipase sequences.

In the present specification, "clade" is a population composed of ancestral sequences in a common ancestor, and sequences of all descendants derived from the common ancestor (existing sequences and intermediate ancestral sequences between the existing sequences and the ancestral sequences in the common ancestor) (evolution.berkeley.edu/evolibrary/article/0_0_0/evo_06). The clade can be visualized as a phylogenetic tree. A subgroup forming a group within a clade in the phylogenetic tree is referred to as a subclade, and sequences in a subclade are more closely related to each other than sequences in a different subclade of the same clade.

In the present specification, "QGLP clade" refers to a clade composed of a sequence group of lipases belonging to subfamily I.1 of bacterial lipases, and lipases closely related to the lipases belonging to subfamily I.1.

Lipase sequences belonging to the QGLP clade can be defined as sequences containing a motif set forth in [QH][GA][LVMI][PSAQ]XX[WRPC][GCDR][GESAD]XG (SEQ ID NO: 57). Here, X represents any amino acid residue. Mutants whose parent sequences have the same motif may also be included in the lipase sequences belonging to the QGLP clade.

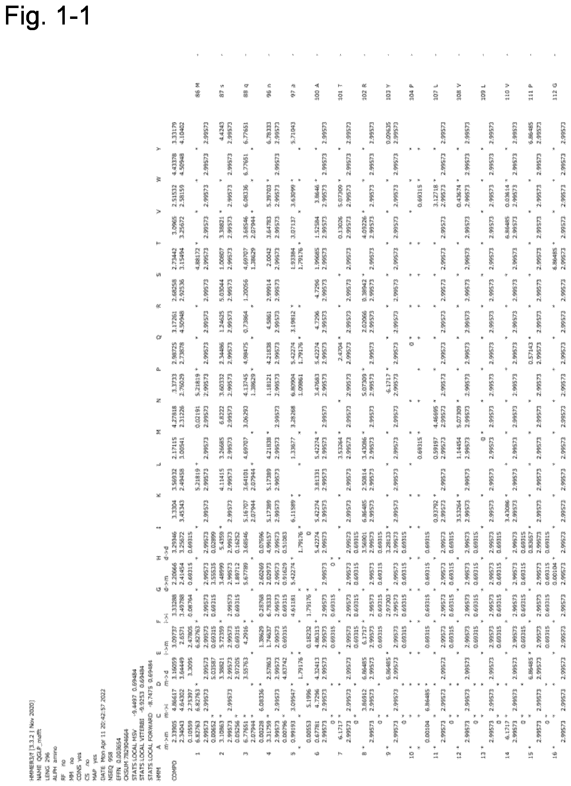

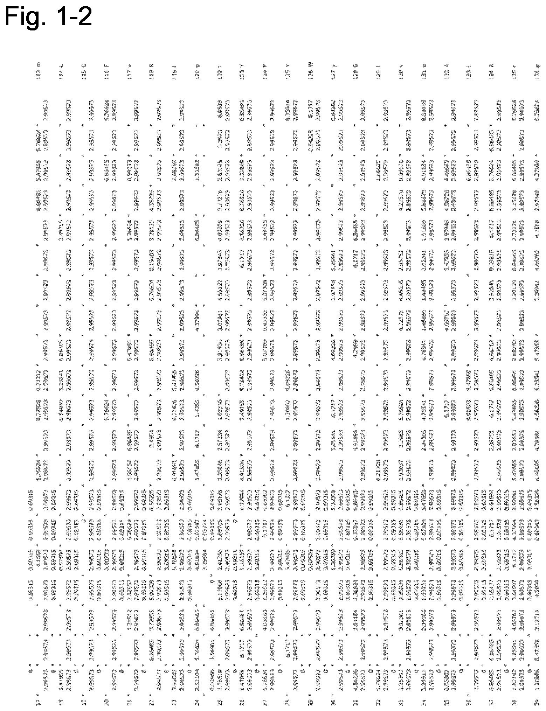

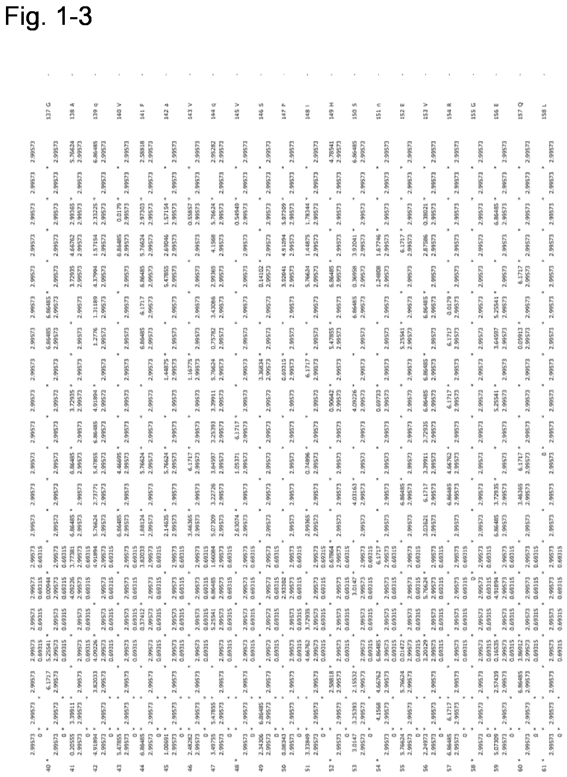

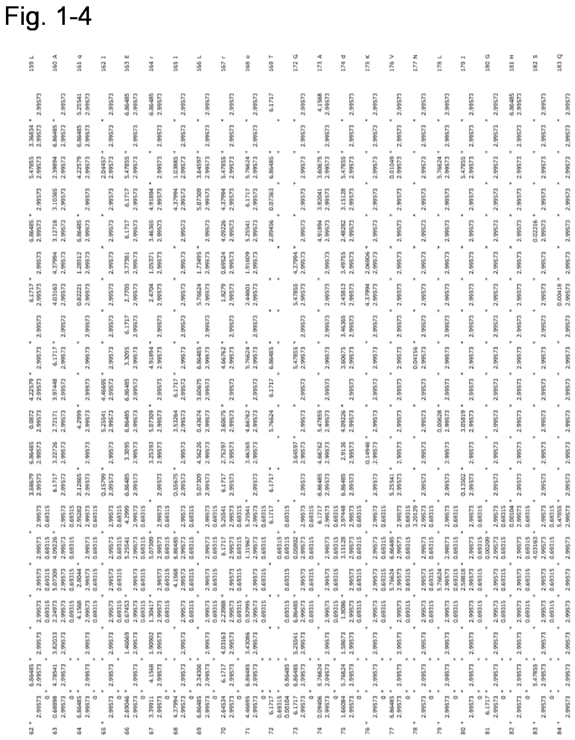

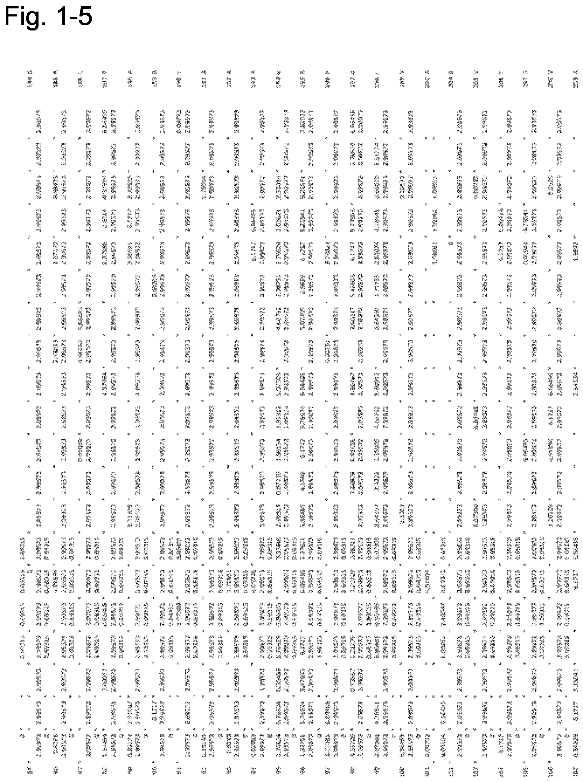

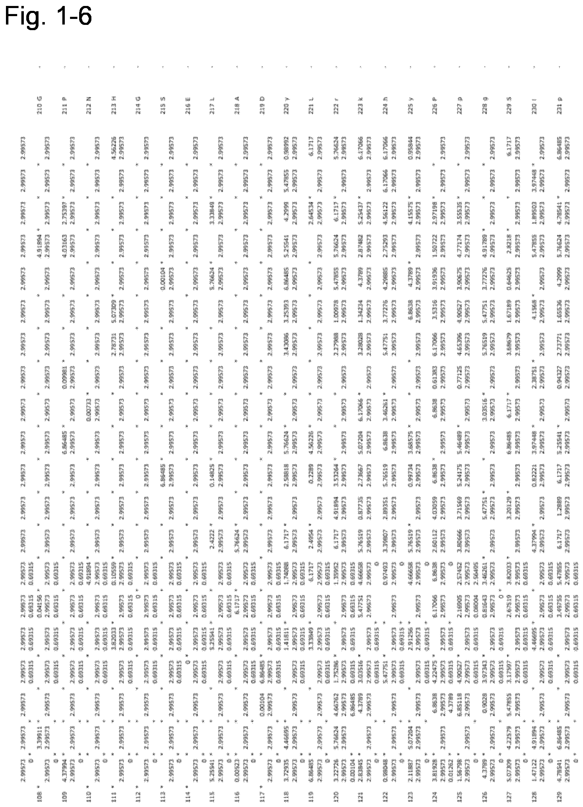

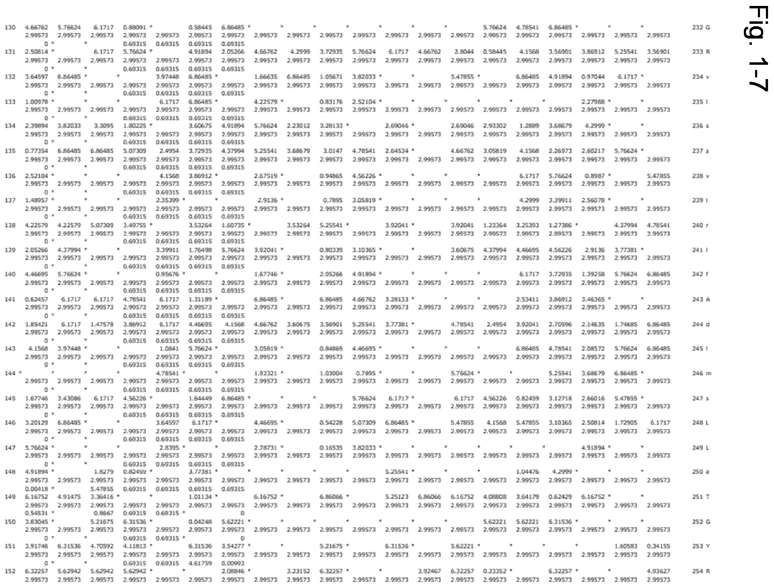

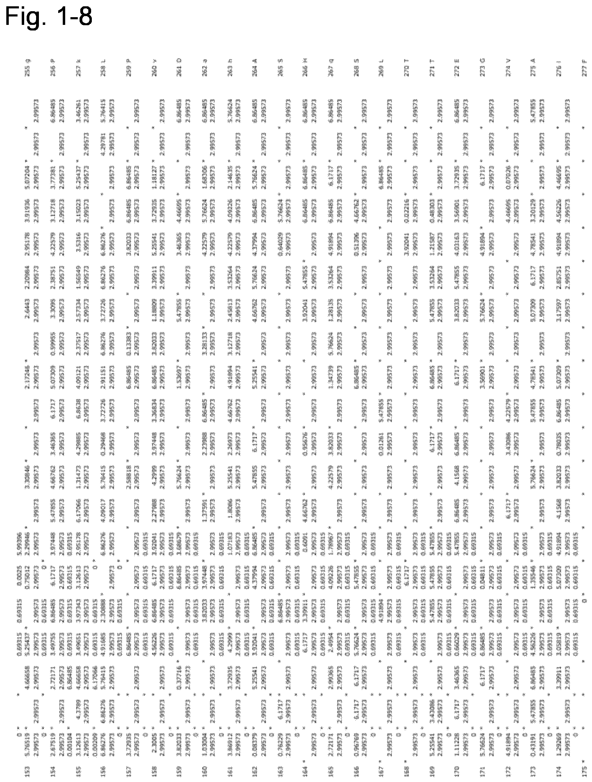

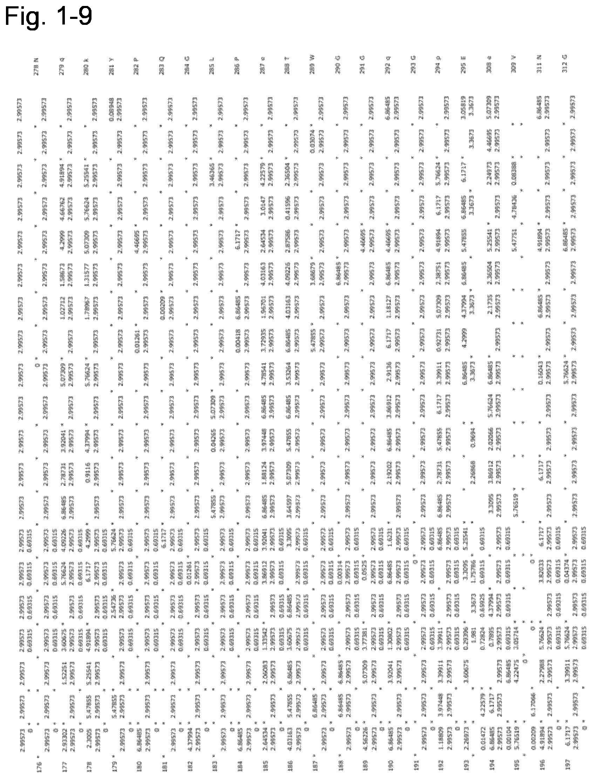

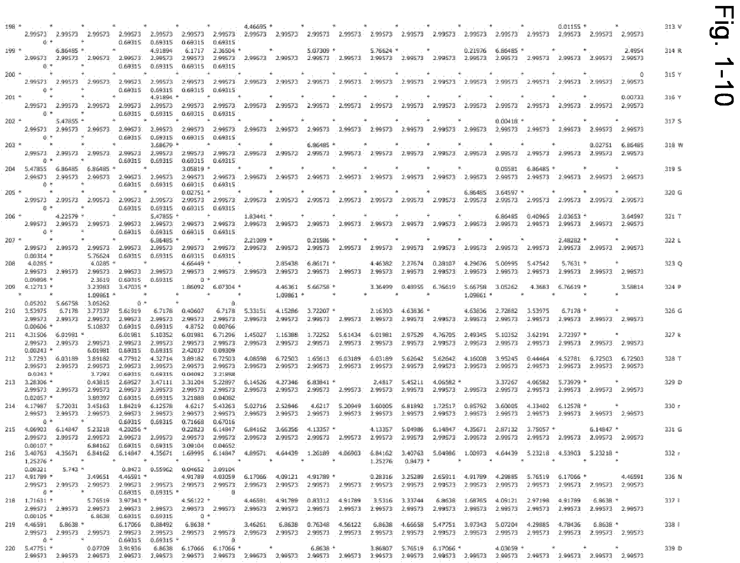

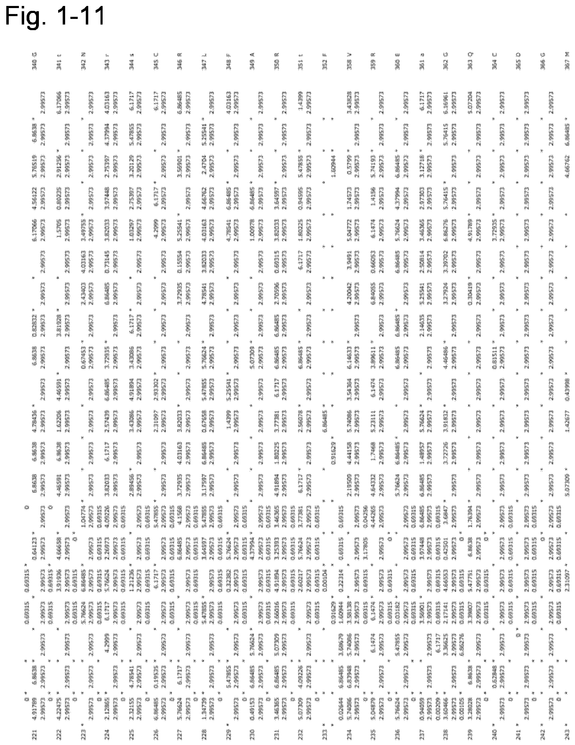

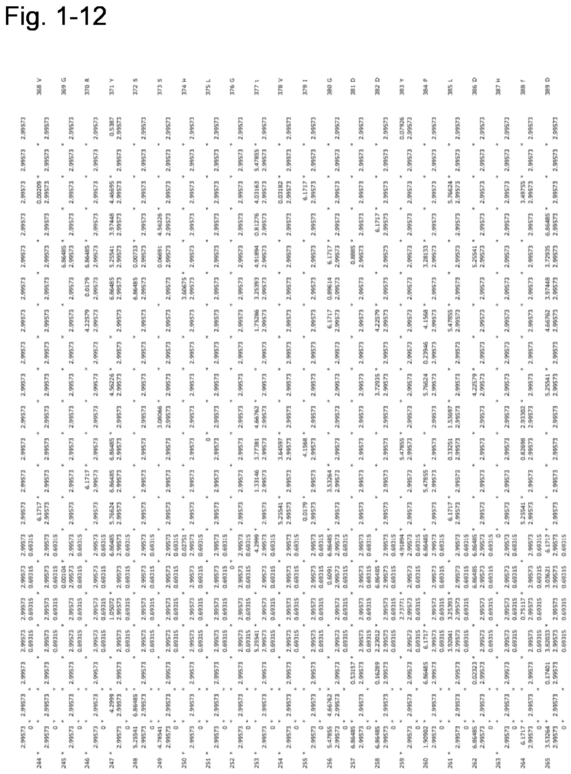

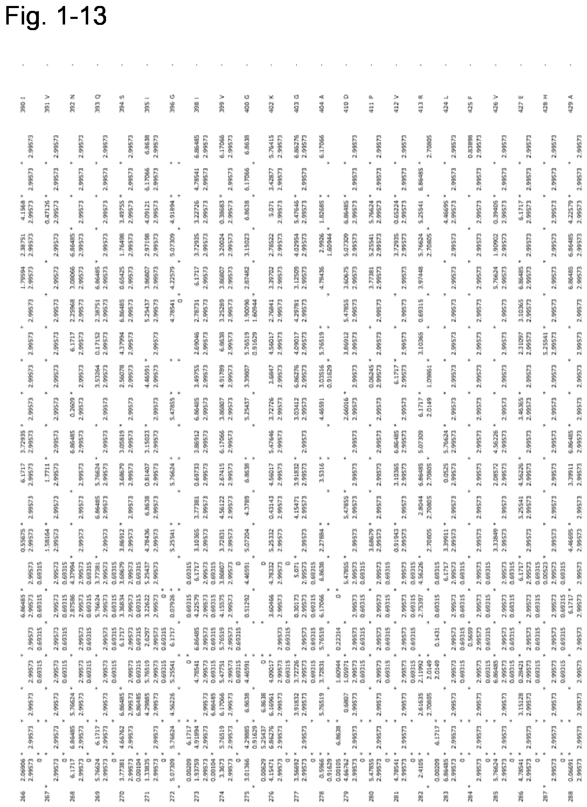

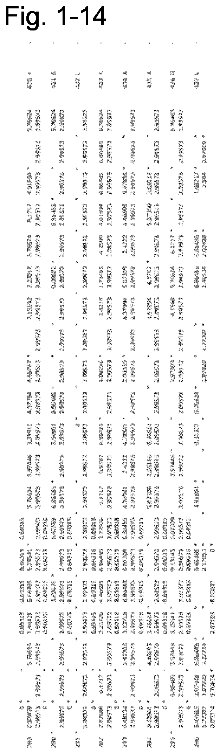

Alternatively, the lipase sequences belonging to the QGLP clade can also be defined as sequences which have an hidden Markov model (HMM) score of 350 or more in full length relative to the HMM profiles shown in Fig. 1. The HMM profiles of Fig. 1 were generated based on multiple alignments constructed with the default parameters of MAFFT v7.471 using a lipase sequence group belonging to the QGLP clade. For the HMM generation from the multiple alignments, HMMER version 3.3.2 (http://hmmer.org/) was used, and --pnone --wnone was specified as the parameter. The HMM profiles are binarized using hmmpress, and a homology search is performed using hmmscan, whereby the HMM score of any sequence relative to the generated HMM profiles of the QGLP clade can be calculated. Table 4 below shows the NCBI accession numbers of the lipase sequence group used to generate the HMM profiles, which define the QGLP clade.

The lipase sequences belonging to the QGLP clade preferably do not contain secretion signals. Secretion signal sequences refer to signal sequences in the Sec secretion pathway. The presence or absence of Sec secretion signals can be determined, for example, using a signal prediction tool, such as SignalP-5.0 (https://services.healthtech.dtu.dk/service.php?SignalP-5.0).

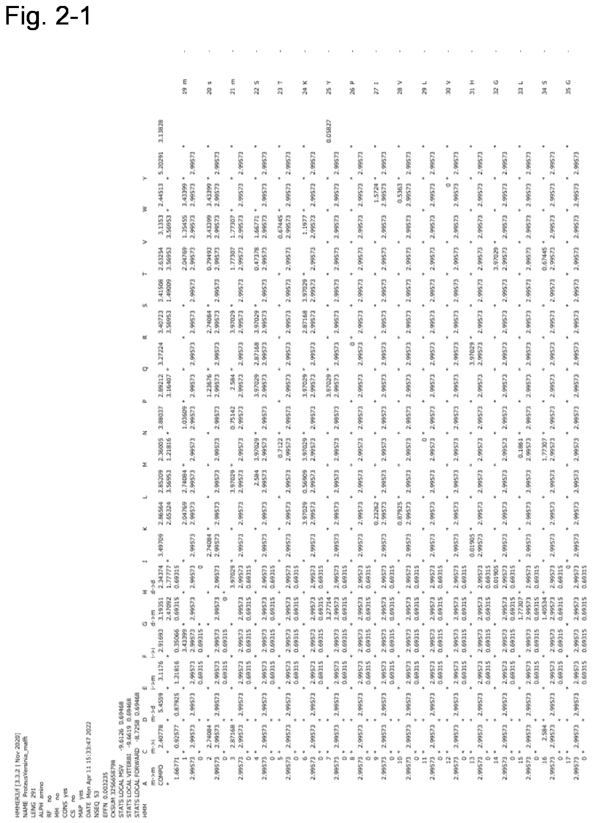

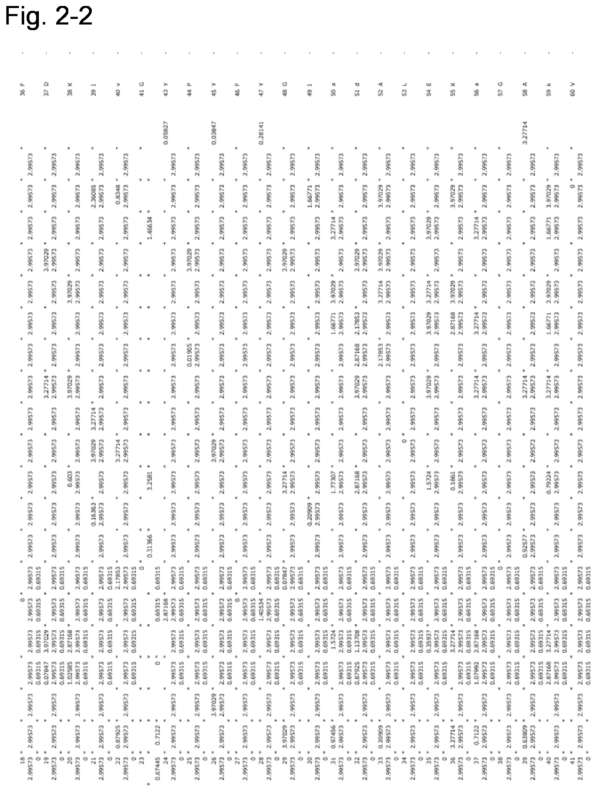

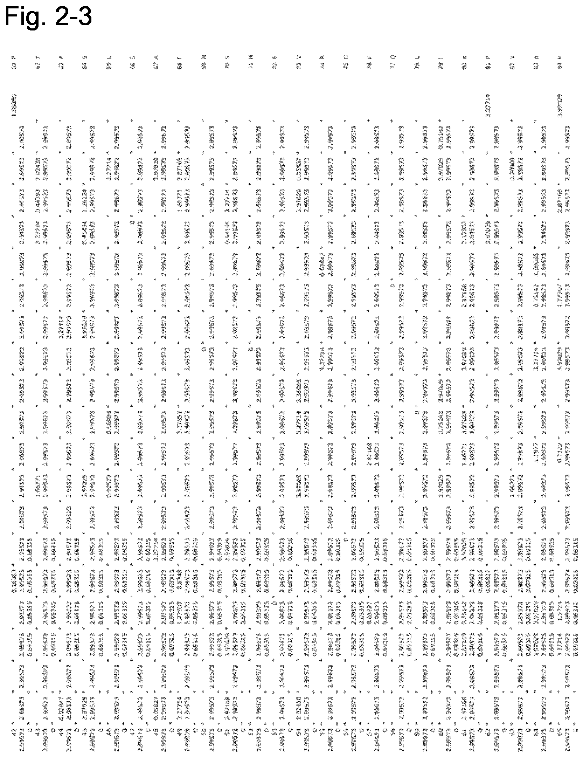

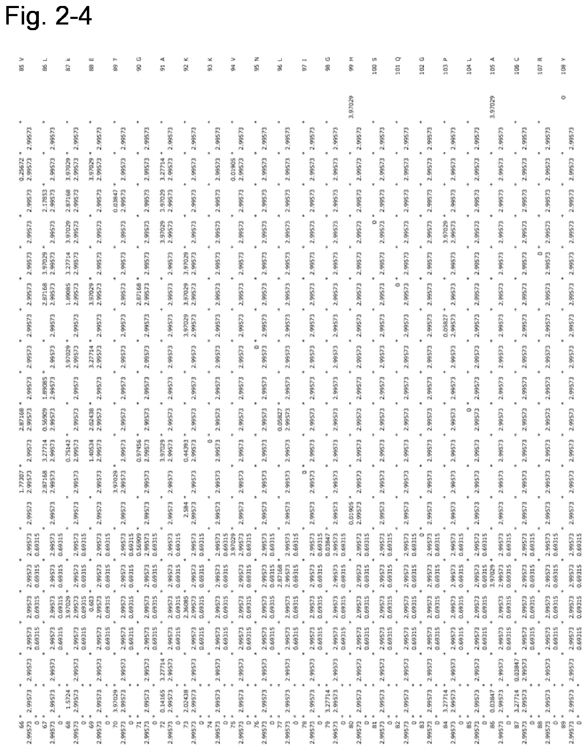

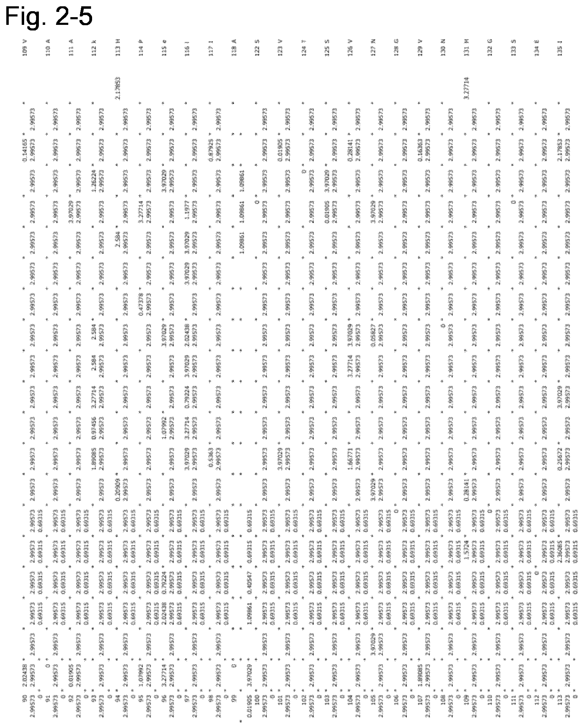

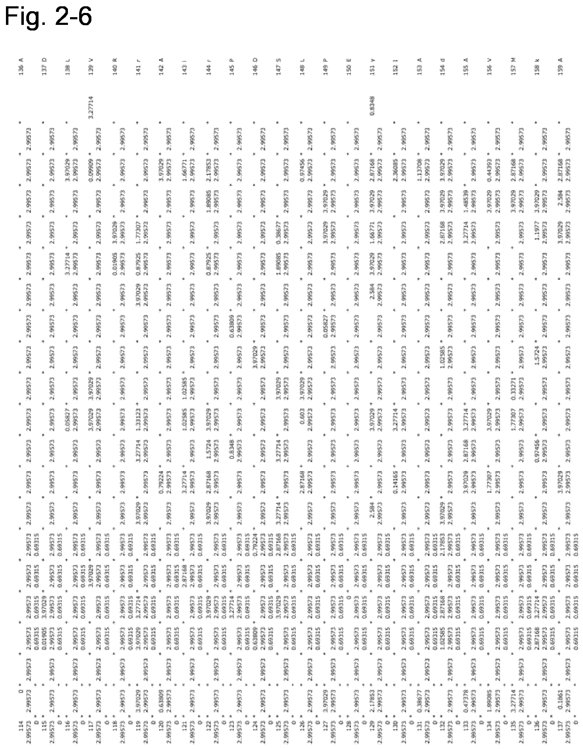

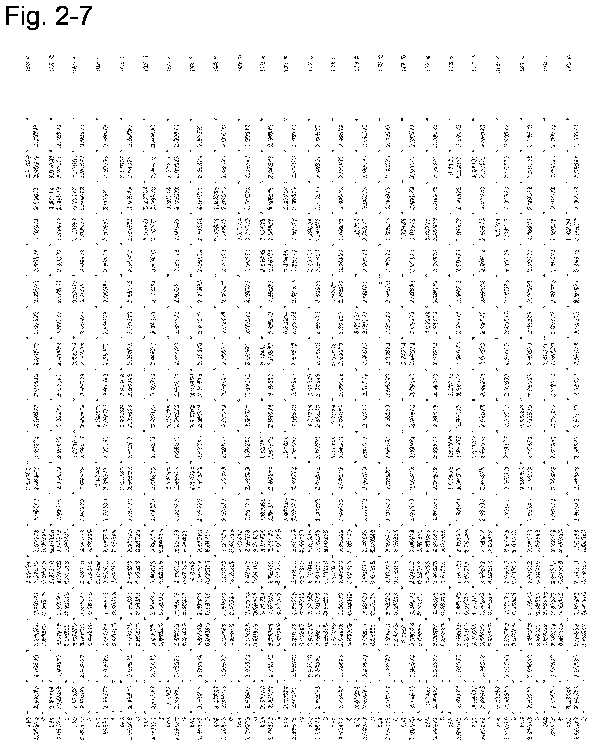

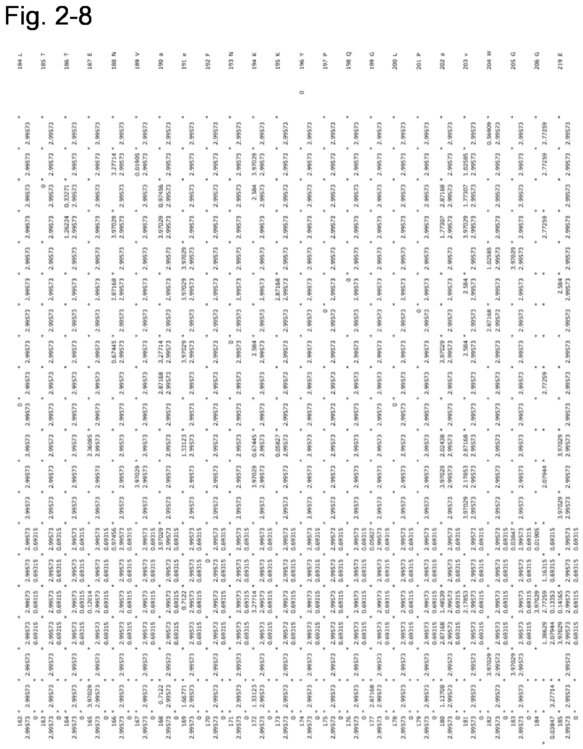

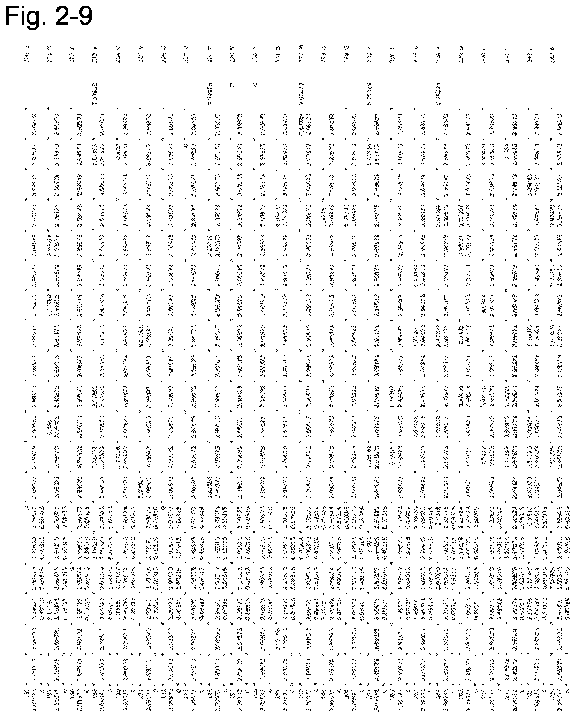

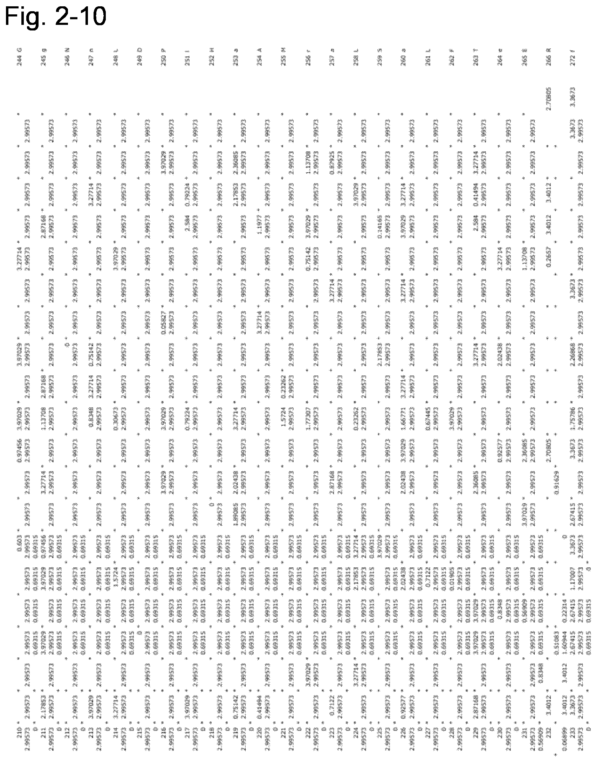

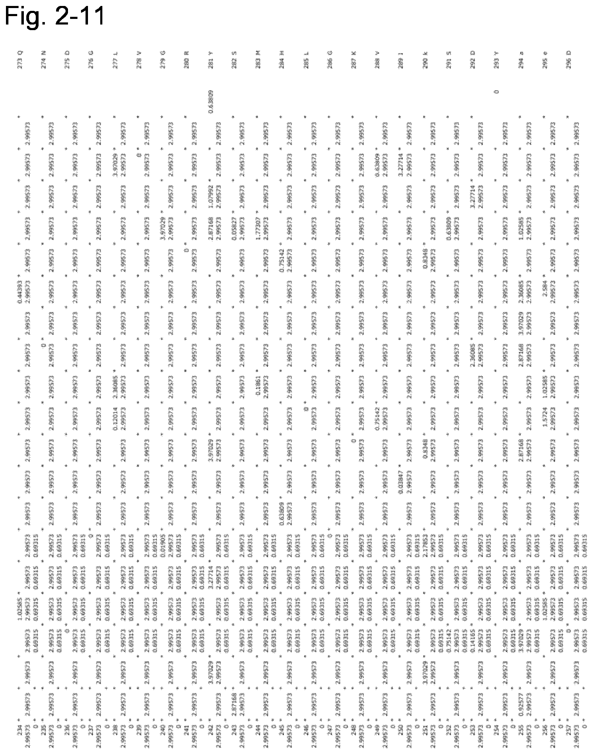

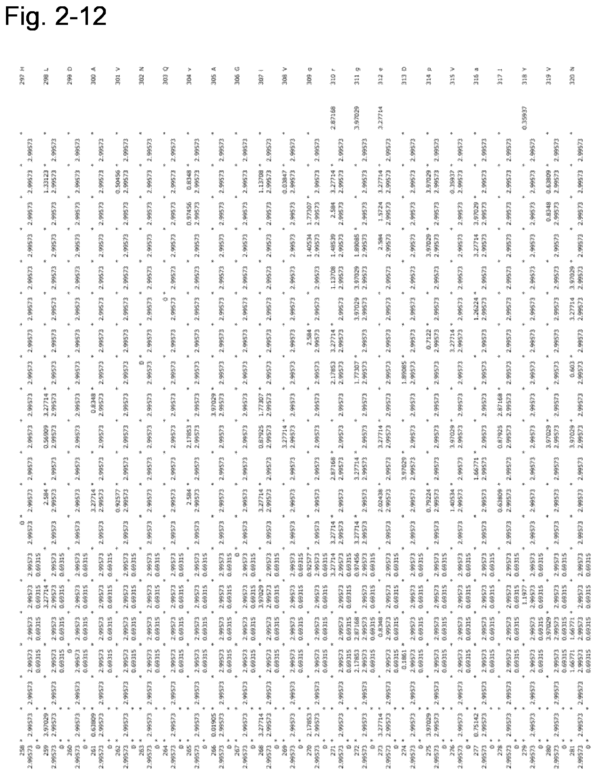

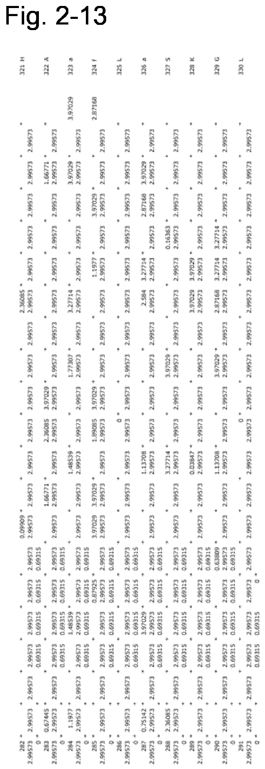

In the present specification, *"Proteus*/*Yersinia* clade" refers to a subclade of the QGLP clade, and includes lipase sequences derived from *Proteus* and *Yersinia* bacteria. The lipase sequences belonging to the *Proteus*/*Yersinia* clade can be defined as sequences which have an HMM score of 600 or more in full length relative to the HMM profiles shown in Fig. 2. The HMM profiles of Fig. 2 were generated based on multiple alignments constructed with MAFFT v7.471 using a lipase sequence group belonging to the *Proteus*/*Yersinia* clade. The parameter used was --localpair --maxiterate 16. For the HMM generation from the multiple alignments, HMMER version 3.3.2 was used, and --pnone --wnone was specified as the parameter. The HMM profiles are binarized using hmmpress, and a homology search is performed using hmmscan, whereby the HMM score of any sequence relative to the generated HMM profiles of the *Proteus*/*Yersinia* clade can be calculated. Table 6 below shows the NCBI accession numbers of the lipase sequence group used to generate the HMM profiles of the *Proteus*/*Yersinia* clade.

In the present specification, the identity between amino acid sequences is calculated by the Lipman-Pearson method (Science, 1985, 227: 1435-1441). Specifically, the identity is calculated by analysis using a homology analysis (Search homology) program of genetic information processing software GENETYX Ver. 12 at a unit size to compare (ktup) of 2.

In the present specification, the "position corresponding to" a position in an amino acid sequence can be determined by arranging (aligning) the target sequence and the reference sequence so as to give maximum homology. The alignment of amino acid sequences can be performed by using known algorithms, and its procedure is well known to a person skilled in the art. For example, the alignment can be performed by using the Clustal W multiple alignment program (Thompson, J.D., et al., 1994, Nucleic Acids Res. 22: 4673-4680) with default settings. Alternatively, Clustal W2 and Clustal omega, which are revised versions of Clustal W, can also be used. Clustal W, Clustal W2, and Clustal omega are available, for example, on the following websites: Clustal website operated by University College Dublin [www.clustal.org], European Bioinformatics Institute (EBI, [www.ebi.ac.uk/index.html]), and the DNA Data Bank of Japan (DDBJ, [www.ddbj.nig.ac.jp/searches-j.html]) operated by the National Institute of Genetics. The position in the target sequence aligned to any position in the reference sequence by the above-described alignment is regarded as the "position corresponding to" that position.

In the present specification, the "operable linkage" between a regulatory region, such as a promoter, and a gene means that the gene and the regulatory region are linked so that the gene can be expressed under the control of the regulatory region. Procedures for the "operable linkage" between the gene and the regulatory region are well known to a person skilled in the art.

In the present specification, the "upstream" and "downstream" relating to a gene refer to the upstream and downstream of the gene in the transcription direction. For example, "a gene located downstream of a promoter" means that the gene is present on the 3' side of the promoter in the DNA sense strand, and the upstream of a gene means a region on the 5' side of the gene in the DNA sense strand.

It is known that the activity of lipases is inhibited by a surfactant contained in cleansing compositions. In fact, as verified by the present inventors, known lipases TLL, Lipr139, PvLip, and Lipr138, whose suitability for cleansing is disclosed, were all received large activity inhibition by the coexistence with a surfactant. Activity inhibition by a surfactant is a major issue common in lipases for cleansing. Further, lipases are often used together with a surfactant not only for cleansing applications, but also for industrial applications such as pulp processing, and activity inhibition by a surfactant is an issue for the entire industrial lipases. Accordingly, there is a demand for a method for efficiently searching for lipases which have resistance to activity inhibition by a surfactant and/or show excellent detergency in the presence of a surfactant.

As a result of careful consideration in light of these issues, the present inventors found that lipases having at least one of resistance to activity inhibition by a surfactant and excellent detergency in the presence of a surfactant can be efficiently obtained by designing lipase ancestral sequences by ancestral sequence reconstruction.

In general, ancestral enzymes are known to exhibit characteristics such as high heat resistance and high optimal temperature; however, characteristics related to activity inhibition by a surfactant and characteristics related to cleansing action at low temperatures have been completely unknown until now. The above results were unexpected.

The search method of the present invention can be used to efficiently obtain lipases having at least one of resistance to activity inhibition by a surfactant and excellent detergency in the presence of a surfactant.

As shown in the examples provide later, ancestral lipases consisting of ancestral sequences designed by ancestral sequence reconstruction using a lipase existing sequence group could be heterologously expressed with a higher probability than existing lipases, well retained lipase activity even in the presence of a surfactant, i.e., had resistance to activity inhibition by a surfactant, and showed excellent detergency even in the presence of a surfactant. When such ancestral lipases were ancestral lipases consisting of ancestral sequences belonging to the QGLP clade, particularly ancestral lipases consisting of ancestral sequences belonging to the QGLP clade and ancestral lipases consisting of ancestral sequences of lipase existing sequences belonging to the *Proteus*/*Yersinia* clade, they had resistance to activity inhibition by a surfactant with a further higher probability, and showed excellent detergency even in the presence of a surfactant. Therefore, when lipase ancestral sequences are designed by ancestral sequence reconstruction using a lipase sequence group, ancestral lipases consisting of the ancestral sequences are lipases which show at least one of resistance to activity inhibition by a surfactant and excellent detergency in the presence of a surfactant with a higher probability than existing lipases. In other words, it is possible to efficiently search for lipases which show at least one of resistance to activity inhibition by a surfactant and excellent detergency in the presence of a surfactant.

Therefore, the present invention provides a lipase search method comprising the steps of: designing lipase ancestral sequences by ancestral sequence reconstruction using a lipase sequence group; measuring at least one of a lipase activity of lipases consisting of the selected ancestral sequences in the presence of a surfactant and a detergency thereof in the presence of a surfactant; and comparing measured results with at least one of a lipase activity of a standard lipase in the presence of a surfactant and a detergency thereof in the presence of a surfactant.

The lipase sequence group used in the method of the present invention is a lipase existing sequence group, and its origin is not particularly limited; however, preferred is a prokaryote-derived bacterial lipase sequence group, more preferred is a sequence group containing at least one lipase sequence belonging to subfamily I.1 or subfamily I.2 of bacterial lipases, further more preferred is a lipase sequence group belonging to subfamily I.1 or subfamily I.2 of bacterial lipases, and further more preferred is a lipase sequence group belonging to the QGLP clade.

The number of lipase sequences contained in the lipase sequence group may be at least 2, preferably 10 or more, more preferably 50 or more, and further more preferably 100 or more. The upper limit of the number of lipase sequences is not particularly limited and may be appropriately set. The number of lipase sequences contained in the lipase sequence group is, for example, from 2 to 1 000, from 10 to 1 000, from 50 to 1 000, from 100 to 1 000, from 2 to 3 000, from 10 to 3 000, from 50 to 3 000, or from 100 to 3 000.

"Designing lipase ancestral sequences" means determining the amino acid sequences of ancestral lipases. The lipase ancestral sequences can be designed by ancestral sequence reconstruction, specifically by obtaining lipase existing sequences from a public database or the like, using the obtained lipase existing sequence group to create multiple alignments and then a rooted phylogenetic tree, and estimating the amino acid sequences of ancestral lipases using the ASR program.

General methods can be used for the design of the lipase ancestral sequences. For example, it is possible to use the methods described in Merkl R., Sterner R., Biol Chem. 2016; 397 (1): 1-21, Scossa F., Fernie AR., Comput Struct Biotechnol J. 2021; 19: 1579-1594, and the like. It is also possible to use the general phylogenetic analysis method described in JSBi Bioinformatics Review, 2 (1), 30-57 (2021). A data set of lipase existing sequences can be obtained, for example, from public databases, such as NCBI and UniProtKB, by using BLAST (Altschul et al., 1990). Alternatively, the data set can also be obtained all at once as a family from domain databases, such as InterPro and Pfam. For the creation of multiple alignments, for example, it is possible to use a program, such as Clustal X and Clustal W (Larkin et al., 2007), MUSCLE (Edgar, 2004), MAFFT (Katoh and Standley, 2013), MAFFT-DASH (Rozewicki et al., 2019), PRANK (Loytynoja, 2014), and T-COFFEE (Notredame et al., 2000). The created multiple alignments are appropriately trimmed using a program, such as GBLOCKS (Castresana, 2000) and trimAl (Capella-Gutierrez et al., 2009), and then used to estimate the phylogenetic tree. Most parsimony, Bayesian, and maximum likelihood methods can be used for the estimation of the phylogenetic tree. For example, it is possible to use a program, such as MrBayes (Ronquist et al., 2012), BEAST (Bouckaert et al., 2019), FastTree (Price et al., 2010), PhyML (Guindon et al., 2010), RAxML (Stamatakis et al., 2014), and IQ-TREE (Nguyen et al., 2015). An appropriate evolutionary model used to estimate the phylogenetic tree can be selected by using a program, such as ModelTest-NG (Darriba, D. et al., 2020) and ModelFinder (Kalyaanamoorthy et al., 2017). Dedicated programs can be used for the design of the ancestor sequences, and it is possible to use a program, such as FastML (Pupko et al., 2000), ProtASR2 (Arenas M and Bastolla, 2020), and GRASP (Gabriel et al., 2019). In addition, some phylogenetic tree estimation programs include ancestor sequence design as part of the functionality thereof, and it is also possible to use programs, such as PAML (Yang, 1997), RAxML, and IQ-TREE. MEGA (Tamura et al., 2021) can also be used as an integrated environment for designing ancestral sequences all at once from these phylogenetic analyses.

The designed lipase ancestral sequences form a search population. The number of lipase ancestral sequences designed, i.e., the search population size, is not particularly limited, and may be appropriately set by a person skilled in the art. The number of lipase ancestral sequences designed can vary depending on, for example, the number of sequences contained in the lipase sequence group subjected to the ancestral sequence determination method, and which branching point on the created rooted phylogenetic tree the ancestral sequences are designed by going back to.

In the method of the present invention, the lipase ancestral sequences designed by ancestral sequence reconstruction as described above may be modified to design modified ancestral sequences, and the modified ancestral sequences may be added to the search population. In this case, the modified ancestral sequences may be treated in the same manner as for the ancestral sequences in the subsequent steps of the method of the present invention. Examples of modification include substitution, deletion, addition, or insertion of one or several amino acid residues in the ancestral sequences or chimerization of the ancestral sequences, and preferably substitution of one or several amino acid residues in the ancestral sequences or chimerization of the ancestral sequences. "One or several" can mean preferably from 1 to 20, more preferably from 1 to 10, further more preferably from 1 to 5, further more preferably from 1 to 3, and further more preferably 1 or 2. The "addition" of amino acid residues includes addition of one or several amino acid residues to one end and both ends of the sequence. The chimerization of ancestral sequences means that part of an ancestral sequence is replaced with an amino acid sequence at a position corresponding to that part in another ancestral sequence or an existing sequence. The number of sequences used for chimerization is at least 2 including the ancestral sequence to be chimerized, and can be preferably from 2 to 11, more preferably from 2 to 6, further more preferably from 2 to 4, and further more preferably 2 or 3. The number of positions of chimerization can be at least 1, preferably from 1 to 10, more preferably from 1 to 5, further more preferably from 1 to 3, and further more preferably 1 or 2. The specific procedure of modification includes producing a modified ancestral sequence obtained by substitution, deletion, addition, or insertion of one or several amino acid residues in a lipase ancestral sequence, or producing a modified ancestral sequence by replacing part of a lipase ancestral sequence with an amino acid sequence at a position corresponding to that part in another ancestral sequence or an existing sequence, or combining them. The modified ancestral sequence can be an amino acid sequence having at least 60% identity to the ancestral sequence before modification. Here, "at least 60% identity" refers to 60% or more, preferably 70% or more, more preferably 80% or more, further more preferably 90% or more, further more preferably 95% or more, and further more preferably 98% or more identity.

In a preferred embodiment, ancestral sequences belonging to the QGLP clade are selected from the designed lipase ancestral sequences. Whether a lipase ancestral sequence belongs to the QGLP clade can be determined based on the presence or absence of the motif set forth in SEQ ID NO: 57 in the ancestral sequence. Specifically, when an ancestral sequence contains the motif set forth in SEQ ID NO: 57, the ancestral sequence can be determined to belong to the QGLP clade. On the other hand, when an ancestral sequence does not contain the motif set forth in SEQ ID NO: 57, the ancestral sequence can be determined not to belong to the QGLP clade. Alternatively, whether an ancestral sequence belongs to the QGLP clade can be determined based on the HMM score of the ancestral sequence relative to HMM profiles created using the lipase sequence group in Table 4 below, i.e., the HMM profiles of the QGLP clade shown in Fig. 1. Specifically, when the HMM score of the full length of an ancestral sequence relative to the HMM profiles of the QGLP clade shown in Fig. 1 is 350 or more, the ancestral sequence can be determined to belong to the QGLP clade. On the other hand, when the HMM score of the full length of an ancestral sequence relative to the HMM profiles of the QGLP clade shown in Fig. 1 is less than 350, the ancestral sequence can be determined not to belong to the QGLP clade. In the present embodiment, ancestral sequences which have been determined to belong to the QGLP clade based on any of the above criteria are selected.

The ancestral sequences belonging to the QGLP clade are preferably sequences corresponding to the ancestral sequences of lipase existing sequences belonging to the *Proteus*/*Yersinia* clade, in terms of search efficiency. The phrase that "the lipase ancestral sequences belonging to the QGLP clade correspond to the ancestral sequences of lipase existing sequences belonging to the *Proteus*/*Yersinia* clade" means that existing sequences which are descendants of the ancestral sequences belonging to the QGLP clade on the rooted phylogenetic tree include at least one lipase existing sequence belonging to the *Proteus*/*Yersinia* clade. Whether a lipase existing sequence belongs to the *Proteus*/*Yersinia* clade can be determined based on the HMM score of the existing sequence relative to HMM profiles created using the lipase sequence group in Table 6 below, i.e., the HMM profiles of the *Proteus*/*Yersinia* clade shown in Fig. 2. Specifically, when the HMM score of the full length of an existing sequence relative to the HMM profiles of the *Proteus*/*Yersinia* clade shown in Fig. 2 is 600 or more, the existing sequence can be determined to belong to the *Proteus*/*Yersinia* clade. On the other hand, when the HMM score of the full length of an existing sequence relative to the HMM profiles of the *Proteus*/*Yersinia* clade shown in Fig. 2 is less than 600, the existing sequence can be determined not to belong to the *Proteus*/*Yersinia* clade.

A lipase consisting of the designed lipase ancestral sequence or a lipase consisting of the designed and selected lipase ancestral sequence (ancestral lipase) can be produced, for example, by expressing the polynucleotide encoding the ancestral lipase.
Preferably, the ancestral lipase can be produced from a transformant into which the polynucleotide encoding the ancestral lipase is introduced. For example, the polynucleotide encoding the ancestral lipase, or a vector containing the polynucleotide, is introduced into a host to obtain a transformant, the transformant is then cultured in a suitable culture medium, and as a result, the ancestral lipase is produced from the polynucleotide encoding the ancestral lipase introduced into the transformant. The produced ancestral lipase can be isolated or purified from the culture to thereby obtain the ancestral lipase.

The polynucleotide encoding the ancestral lipase can be in the form of single- or double-stranded DNA, RNA, or an artificial nucleic acid, or may be cDNA or chemically synthesized intron-free DNA.

The polynucleotide encoding the ancestral lipase can be synthesized chemically or genetically based on the amino acid sequence of the ancestral lipase. For example, the polynucleotide can be chemically synthesized based on the amino acid sequence of the ancestral lipase. For the chemical synthesis of the polynucleotide, nucleic acid synthesis contract services (e.g., provided by Medical & Biological Laboratories Co., Ltd., GenScript, and the like) can be used. Further, the synthesized polynucleotide can be amplified by PCR, cloning, or the like.

The polynucleotide encoding the ancestral lipase can be incorporated into a vector. The type of the vector into which the polynucleotide is to be incorporated is not particularly limited, and any vector, such as a plasmid, phage, phagemid, cosmid, virus, YAC vector, or shuttle vector, may be used. The vector is not limited, but is preferably a vector which can be amplified in bacteria, preferably *Bacillus* bacteria (e.g., *Bacillus subtilis* or mutant strains thereof), and more preferably an expression vector which can induce the expression of transgenes in *Bacillus* bacteria. Among these, shuttle vectors, which are vectors replicable in *Bacillus* bacteria and any other organisms, can be preferably used in the recombinant production of the ancestral lipase. Examples of preferred vectors include, but are not limited to, shuttle vectors, such as pHA3040SP64, pHSP64R, or pASP64 (JP-B-3492935), pHY300PLK (an expression vector which can transform both *Escherichia coli* and *Bacillus subtilis*; Jpn J Genet, 1985, 60: 235-243), pAC3 (Nucleic Acids Res, 1988, 16: 8732); plasmid vectors which can be used in the transformation of Bacillus bacteria, such as pUB110 (J Bacteriol, 1978, 134: 318-329), and pTA10607 (Plasmid, 1987, 18: 8-15); and the like. *Escherichia coli*-derived plasmid vectors (e.g., pET22b(+), pBR322, pBR325, pUC57, pUC118, pUC119, pUC18, pUC19, and pBluescript, and the like) can also be used.

The above vector may contain a DNA replication initiation region or a DNA region containing a replication origin. Alternatively, in the above vector, a regulatory sequence, such as a promoter region for initiating the transcription of the gene, a terminator region, or a secretion signal region for extracellularly secreting the expressed protein, may be operably linked to the upstream of the polynucleotide encoding the ancestral lipase (i.e., ancestral lipase gene).

The type of regulatory sequence, such as a promoter region, a terminator region, or a secretion signal region mentioned above, is not particularly limited, and generally used promoters and secretion signal sequences can be appropriately selected depending on the host into which the vector is introduced. Examples of preferred regulatory sequences that can be incorporated into the vector include the promoter, secretion signal sequence, and the like of the cellulase gene of *Bacllus* sp. KSM-S237 strain.

Alternatively, a marker gene (e.g., a gene resistant to drugs, such as ampicillin, neomycin, kanamycin, and chloramphenicol) for selecting the host into which the above vector is appropriately introduced may be further incorporated into the vector. Alternatively, when an auxotrophic strain is used as the host, a gene an enzyme that synthesize a required nutrient may be incorporated as a marker gene into the vector. Alternatively, when a selection culture medium that requires a specific metabolism for growth is used a gene related to the metabolism may be incorporated as a marker gene into the vector. Examples of such metabolism-related gene include acetamidase genes for utilizing acetamide as a nitrogen source.

The polynucleotide encoding the ancestral lipase, a regulatory sequence, and a marker gene can be linked by a method known in the art, such as SOE (splicing by overlap extension)-PCR (Gene, 1989, 77: 61-68). Procedures for introducing the linked fragment into the vector are well known in the art.

The transformed cell can be obtained by introducing a vector containing the polynucleotide encoding the ancestral lipase into a host, or by introducing a DNA fragment containing the polynucleotide encoding the ancestral lipase into the genome of the host.

Examples of the host cells include microorganisms, such as bacteria and filamentous fungi. Examples of bacteria include *Escherichia coli* and bacteria belonging to the genera *Staphylococcus, Enterococcus, Listeria,* and *Bacillus.* Preferred among these are *Escherichia coli* and *Bacillus* bacteria (e.g., *Bacillus subtilis* Marburg No. *168 (Bacillus subtilis* 168 strain) or mutant strains thereof). Examples of *Bacillus subtilis* mutant strains include the nine-protease-deficient strain KA8AX described in J. Biosci. Bioeng., 2007, 104(2): 135-143, and the eight-protease-deficient strain with improved protein folding efficiency, D8PA strain, described in Biotechnol. Lett., 2011, 33(9): 1847-1852. Examples of filamentous fungi include Trichoderma, Aspergillus, Rhizopus, and the like.

Methods commonly used in the art, such as the protoplast method and the electroporation method, can be used as the method for introducing the vector into the host. Strains with appropriate introduction are selected using marker gene expression, auxotrophy, and the like as indices, whereby the target transformant into which the vector is introduced can be obtained.

Alternatively, a fragment in which the polynucleotide encoding the ancestral lipase, a regulatory sequence, and a marker gene are linked can also be introduced directly into the genome of the host. For example, a DNA fragment in which sequences complementary to the genome of the host are added to both ends of the linked fragment is constructed by the SOE-PCR method or the like, and this DNA fragment is then introduced into the host to induce homologous recombination between the host genome and the DNA fragment, whereby the polynucleotide encoding the ancestral lipase is introduced into the genome of the host.

When a transformant into which the polynucleotide encoding the ancestral lipase, or a vector containing the polynucleotide is introduced, is cultured in a suitable culture medium, the gene encoding the protein on the vector is expressed to produce the ancestral lipase. The culture medium used for culturing the transformant can be appropriately selected by a person skilled in the art depending on the type of microorganism of the transformant.

Alternatively, the ancestral lipase may be expressed from the polynucleotide encoding the lipase of the present invention or a transcript thereof using a cell-free translation system. The "cell-free translation system" is an in vitro transcription-translation system or an in vitro translation system constructed by adding reagents, such as amino acids, necessary for the protein translation to a suspension obtained by mechanically destroying a cell, which serves as the host.

The ancestral lipase produced in the above culture or cell-free translation system can be isolated or purified by using general methods used for protein purification, such as centrifugation, ammonium sulfate precipitation, gel chromatography, ion-exchange chromatography, and affinity chromatography, singly or in a suitable combination. The protein collected from the culture may be further purified by known means.

In the method of the present invention, the thus-obtained ancestral lipase is used to measure at least one of a lipase activity of the ancestral lipase in the presence of a surfactant and a detergency thereof in the presence of a surfactant. In existing lipases and ancestral lipases, the lipase activity in the presence of a surfactant is well correlated with the detergency in the presence of a surfactant, and at least one of them can be measured to analogize the other.

The surfactant is not particularly limited and may be appropriately selected depending on the purpose of use of lipases, the conditions of use, and the like. For example, a surfactant contained in a cleansing agent composition into which a lipase is desirably blended can be preferably used. Examples of the surfactant include one or a combination of anionic surfactants, nonionic surfactants, amphoteric surfactants, and cationic surfactants, preferably one or a combination of anionic surfactants and nonionic surfactants, more preferably one or a combination of sulfosuccinic acid esters or salts thereof, SDS, and Triton X-100, and further more preferably one or a combination of SDS and Triton X-100.

Sulfosuccinic acid esters or salts thereof are known as components to be blended into cleansing agent compositions (e.g., JP-A-2019-182911). The sulfosuccinic acid ester or a salt thereof is preferably a sulfosuccinic acid branched alkyl ester having a branched alkyl group having 9 or more and 12 or less carbon atoms or a salt thereof, more preferably a sulfosuccinic acid branched alkyl ester having a branched alkyl group having 9 or 10 carbon atoms or a salt thereof, and further more preferably a sulfosuccinic acid branched alkyl ester having a branched alkyl group having 10 carbon atoms or a salt thereof. Moreover, the sulfosuccinic acid ester or a salt thereof is preferably a sulfosuccinic acid di-branched alkyl ester whose two branched alkyl groups are each a branched alkyl group having 9 or more and 12 or less carbon atoms or a salt thereof, more preferably a sulfosuccinic acid di-branched alkyl ester whose two branched alkyl groups are each a branched alkyl group having 9 or 10 carbon atoms or a salt thereof, further more preferably a sulfosuccinic acid di-branched alkyl ester whose two branched alkyl groups are each a branched alkyl group having 10 carbon atoms or a salt thereof, and further more preferably bis-(2-propylheptyl) sulfosuccinate or a salt thereof.

Examples of salts include alkali metal salts, alkanolamine salts, and the like, preferably alkali metal salts or alkanolamine salts, more preferably a salt selected from the group consisting of a sodium salt, a potassium salt, a triethanolamine salt, a diethanolamine salt, and a monoethanolamine salt, and further more preferably a sodium salt.

The lipase activity of the ancestral lipase in the presence of a surfactant may be measured in accordance with a method well known in the art. For example, the lipase activity of the ancestral lipase in the presence of a surfactant can be measured based on the rate of increase in absorbance associated with the release of 4-nitrophenol by the hydrolysis of 4-nitrophenyl octanoate in the surfactant solution mentioned above containing the ancestral lipase. The activity to be measured may be absolute activity or relative activity; however, it is preferable to measure relative activity while eliminating the influence of activity in the solvent from the viewpoint of comparison of a plurality of lipases. As the lipase activity of the ancestral lipase in the presence of a surfactant is higher, it can be evaluated that the ancestral lipase has higher resistance to lipase activity inhibition by the surfactant. An example of the specific procedure of measuring the lipase activity in the presence of a surfactant is shown below.

### <Measurement of lipase activity in the presence of a surfactant>

20 mM 4-nitrophenyl octanoate (SIGMA) in 20 mM Tris-HCl (pH: 7.0) is used as a substrate solution. The surfactant solution used is prepared by adding 0.1% (w/v) SDS or Triton X-100 to 20 mM Tris-HCl (pH: 7.0). 5 µL of the lipase solution adjusted to 2 ppm and 100 µL of the surfactant solution are mixed in each well of a 96-well assay plate, 10 µL of the substrate solution is added thereto, and the absorbance change (OD/min) at 405 nm is measured at 30°C. The difference ΔOD/min from the blank (enzyme-free sample) is used as the activity value. The activity value of each lipase in the surfactant solution is divided by the activity value when using 20 mM Tris-HCl (pH: 7.0) (activity in the buffer) in place of the surfactant solution, and then multiplied by 100. The resulting value is determined as the relative activity (%).

Alternatively, 20 mM 4-nitrophenyl octanoate in 20 mM Tris-HCl (pH: 7.0) is used as a substrate solution. The aqueous medium shown in Table 1 below is used as a model cleansing liquid. 2 µL of the lipase solution adjusted to 4 ppm and 100 µL of a test liquid (model cleansing liquid or 20 mM Tris-HCl (pH: 7.0)) are mixed in each well of a 96-well assay plate, 10 µL of the substrate solution is added thereto, and the absorbance change (OD/min) at 405 nm is measured at 30°C. The difference ΔOD/min from the blank (enzyme-free sample) is determined. Separately, 10 µL of 20 mM Tris-HCl (pH: 7.0) containing 4-nitrophenol with a known concentration is mixed with 100 µL of each test liquid, the absorbance at 405 nm is measured, and a calibration curve is created. From the calibration curve of each test liquid and the ΔOD/min value, the release rate (µM/min) of 4-nitrophenol per minute is calculated as the activity value. The activity value when using the model cleansing liquid as the test liquid is divided by the activity value when using 20 mM Tris-HCl (pH: 7.0) as the test liquid (activity in the buffer), and then multiplied by 100. The resulting value is determined as the relative activity (%).

"Detergency" means an ability to bring about the removal of stains, particularly oily stains, in the washing or cleansing step. The detergency of the ancestral lipase in the presence of a surfactant may be measured in accordance with a method well known in the art. For example, the detergency of the ancestral lipase in the presence of a surfactant can be measured in such a manner that a cleansing liquid containing the ancestral lipase and the surfactant mentioned above is added to a model stain containing a predetermined indicator substance (e.g., a staining agent highly soluble in fat, such as Sudan III), followed by cleansing treatment under predetermined conditions, a part of the cleansing liquid is taken out, the concentration of the indicator substance in the model stain solubilized in the cleansing liquid by the cleansing treatment is measured, and the difference from the blank can be determined as the index for detergency. The cleansing liquid as mentioned herein may further contain components generally used for cleansing compositions. The temperature during measurement is not particularly limited, but is preferably a low temperature. The "low temperature" as mentioned herein may be 40°C or lower, 35°C or lower, 30°C or lower, or 25°C or lower, and 5°C or higher, 10°C or higher, or 15°C or higher. The "low temperature" may also be from 5 to 40°C, from 10 to 35°C, from 15 to 30°C, or from 15 to 25°C. An example of the specific procedure of measuring detergency is shown below.

### <Measurement of detergency in the presence of a surfactant>

Beef tallow (SIGMA, 03-0660) and rapeseed oil (SIGMA, 23-0450) are mixed at a weight ratio of 9:1, and dissolved in 3 times the amount of chloroform, followed by coloring with 0.2 wt% Sudan III, and the resultant is used as a model stain. 10 µL of the model stain is added dropwise to the bottom of each well of a 96-deep-well polypropylene plate, and chloroform is evaporated and dried to prepare a stain plate. The cleansing liquid used is prepared by adding 1/100 volume of 240 ppm lipase solution to the model cleansing liquid shown in Table 1 below. 300 µL of the cleansing liquid is added slowly to each well of the stain plate, and allowed to stand at room temperature (about 22°C) for 15 minutes to perform immersion cleansing. 100 µL of the cleansing liquid is taken out from each well so as not to be in contact with the stain on the bottom and transferred to a new 96-well plate. In order to quantify Sudan III in the model stain solubilized in the cleansing liquid by immersion cleansing, the absorbance at 500 nm (A500) is measured. A value ΔA500 obtained by subtracting A500 before cleansing from A500 after cleansing corresponds to the amount of oil released in the cleansing liquid, and can be used as an index for detergency.

Then, in the method of the present invention, the measured result of at least one of a lipase activity of the ancestral lipase in the presence of a surfactant and a detergency thereof in the presence of a surfactant is compared with at least one of a lipase activity of a standard lipase in the presence of a surfactant and a detergency thereof in the presence of a surfactant. Preferably, further, based on the comparison results, an ancestral lipase of which at least one of the lipase activity in the presence of a surfactant and the detergency in the presence of a surfactant is further improved than that of the standard lipase is selected. If the lipase activity of an ancestral lipase in the presence of a surfactant is higher than the lipase activity of the standard lipase under the same conditions by preferably 5%, more preferably 10%, and further more preferably 20%, the ancestral lipase can be evaluated as a lipase having resistance to lipase activity inhibition by the surfactant, and the ancestral lipase can be selected as the target lipase. If the detergency of an ancestral lipase in the presence of a surfactant is higher than the detergency of the standard lipase under the same conditions by preferably 5%, more preferably 10%, and further more preferably 20%, the ancestral lipase can be evaluated as a lipase having excellent detergency in the presence of the surfactant, and the ancestral lipase can be selected as the target lipase. If the lipase activity of an ancestral lipase in the presence of a surfactant is higher than the lipase activity of the standard lipase under the same conditions by preferably 5%, more preferably 10%, and further more preferably 20%, and if the detergency of the ancestral lipase in the presence of a surfactant is higher than the detergency of the standard lipase under the same conditions by preferably 5%, more preferably 10%, and further more preferably 20%, the ancestral lipase can be evaluated as a lipase having resistance to lipase activity inhibition by the surfactant and having excellent detergency in the presence of the surfactant, and the ancestral lipase can be selected as the target lipase. The standard lipase as mentioned herein can be a known lipase. Various industrial lipases are known from literatures, and a lipase consisting of an amino acid sequence set forth in SEQ ID NO: 2, 4, 6, or 8 is preferably used as the standard lipase. In a preferred example, the standard lipase is a lipase consisting of the amino acid sequence set forth in SEQ ID NO: 4. Alternatively, the standard lipase may be a model lipase which has certain lipase activity in the presence of a surfactant and certain detergency in the presence of a surfactant. The lipase activity of the model lipase in the presence of a surfactant and the detergency thereof in the presence of a surfactant may be appropriately set based on the performance of a lipase to be searched for. Examples of the model lipase include a lipase whose relative activity is 7% when the lipase activity is measured by the above procedure using SDS as the surfactant, a lipase whose relative activity is 30% when the lipase activity is measured using the above procedure using Triton X-100 as the surfactant, a lipase whose relative activity is 30% when the lipase activity is measured using the above procedure using a model cleansing liquid, and a lipase whose index for detergency ΔA500 is 0.05 when the detergency is measured using the above procedure.

The thus-selected lipase is useful as an enzyme to be blended into various cleansing compositions, and particularly useful as an enzyme to be blended into cleansing compositions suitable for low-temperature cleansing.

Regarding the embodiments described above, the present invention further discloses the following aspects.
<1> A lipase search method comprising steps of:
   designing lipase ancestral sequences by ancestral sequence reconstruction using a lipase sequence group;
   measuring at least one of a lipase activity of lipases consisting of the designed ancestral sequences in the presence of a surfactant and a detergency thereof in the presence of a surfactant; and
   comparing measured results with at least one of a lipase activity of a standard lipase in the presence of a surfactant and a detergency thereof in the presence of a surfactant.
<2> The method according to <1>, further comprising a step of selecting a lipase of which at least one of the lipase activity in the presence of a surfactant and the detergency in the presence of a surfactant is improved more than that of the standard lipase.
<3> The method according to <1> or <2>, wherein the lipase sequence group is a bacterial lipase sequence group, preferably a sequence group comprising at least one lipase sequence belonging to subfamily I.1 or subfamily I.2 of bacterial lipases, more preferably a lipase sequence group belonging to subfamily I.1 or subfamily I.2 of bacterial lipases, and further more preferably a lipase sequence group belonging to the QGLP clade.
<4> The method according to any one of <1> to <3>, further comprising a step of selecting an ancestral sequence belonging to the QGLP clade from the designed ancestral sequences, wherein a lipase consisting of the selected ancestral sequence is to be subjected to the step of measuring at least one of a lipase activity in the presence of a surfactant and a detergency in the presence of a surfactant.
<5> The method according to <4>, wherein the ancestral sequence belonging to the QGLP clade is a sequence comprising a motif set forth in SEQ ID NO: 57, or a sequence having an HMM score of 350 or more in full sequence length relative to the HMM profiles of the QGLP clade shown in Fig. 1.
<6> The method according to <4> or <5>, wherein the ancestral sequence belonging to the QGLP clade corresponds to an ancestral sequence of a lipase existing sequence belonging to the *Proteus*/*Yersinia* clade.
<7> The method according to <6>, wherein the lipase existing sequence belonging to the *Proteus*/*Yersinia* clade is a sequence having an HMM score of 600 or more in full sequence length relative to the HMM profiles of the *Proteus*/*Yersinia* clade shown in Fig. 2.
<8> The method according to any one of <1> to <7>, wherein the surfactant is at least one member selected from the group consisting of a sulfosuccinic acid ester or a salt thereof, SDS, and Triton X-100, and preferably at least one member selected from the group consisting of SDS and Triton X-100.
<9> The method according to any one of <1> to <8>, wherein the standard lipase is a known lipase, preferably a lipase consisting of an amino acid sequence set forth in SEQ ID NO: 2, 4, 6, or 8, and more preferably a lipase consisting of an amino acid sequence set forth in SEQ ID NO: 4.
<10> The method according to any one of <1> to <9>, wherein the detergency is detergency at a low temperature.
<11> The method according to <10>, wherein the detergency is for cleansing at 40°C or lower, 35°C or lower, 30°C or lower, or 25°C or lower, and at 5°C or higher, 10°C or higher, or 15°C or higher, or for cleansing at from 5 to 40°C, from 10 to 35°C, from 15 to 30°C, or from 15 to 25°C.
<12> The method according to any one of <1> to <11>, further comprising steps of:
   modifying the designed ancestral sequences to design modified ancestral sequences;
   measuring at least one of a lipase activity of lipases consisting of the designed modified ancestral sequences in the presence of a surfactant and a detergency thereof in the presence of a surfactant; and
   comparing measured results with at least one of a lipase activity of a standard lipase in the presence of a surfactant and a detergency thereof in the presence of a surfactant.
<13> The method according to <12>, further comprising a step of selecting a lipase of which at least one of the lipase activity in the presence of a surfactant and the detergency in the presence of a surfactant is improved more than that of the standard lipase.
<14> The method according to <12> or <13>, wherein the modifying is amino acid residue substitution, deletion, addition, or insertion in the ancestral sequences or chimerization of the ancestral sequences, and preferably amino acid residue substitution in the ancestral sequences or chimerization of the ancestral sequences.

### Examples

The present invention is described in more detail below based on examples; however, the present invention is not limited thereto.

### Reference Example 1: Evaluation of Lipr139, PvLip, Lipr138, and TLL

### (1) Construction of lipase expression plasmids

Lipase expression plasmids were constructed using the VHH expression plasmid containing a *Bacillus subtilis* spoVG gene-derived promoter described in SEQ ID NO: 26 in WO2021/153129 as a template. Lipase expression constructs linked to spoVG promoter were constructed by introducing each lipase gene into a region corresponding to the full length of the VHH gene of the above plasmid by the In-Fusion reaction. Plasmids pHY-Lipr139, pHY-PvLip, and pHY-Lipr138 were constructed from artificially synthesized lipase genes Lipr139, PvLip, and Lipr138 (polynucleotides of respective SEQ ID NOs: 3, 5, and 7, which respectively encode the amino acid sequences of SEQ ID NOs: 4, 6, and 8), respectively. Similarly for lipase TLL gene with *Bacillus subtilis* amyE gene-derived signal sequence (a polynucleotide of SEQ ID NO: 55, which encodes the amino acid sequence of SEQ ID NO: 56) linked to the N-terminus (a polynucleotide of SEQ ID NO: 1, which encodes the amino acid sequence of SEQ ID NO: 2; hereinafter referred to as amyEsig-TLL), pHY-amyEsig-TLL was constructed by the In-Fusion reaction.

### (2) Preparation of lipase solutions

The lipase expression plasmids were each introduced into a *Bacillus subtilis* strain by the protoplast method, and cultured in 2×L-maltose culture medium (2% tryptone, 1% yeast extract, 1% NaCl, 7.5% maltose, 7.5 ppm manganese sulfate pentahydrate, 0.04% calcium chloride dihydrate, and 15 ppm tetracycline; % denotes (w/v)%) at 30°C for 2 days. Then, the lipase-containing culture supernatant was collected by centrifugation. The culture supernatant was replaced with 10 mM Tris-HCl+0.01% Triton-X100 (pH: 7.0) buffer by dialysis, and the lipase concentration was quantified from the band intensity of SDS-PAGE.

### (3) Measurement of activity in surfactant solution

4-Nitrophenyl octanoate (SIGMA) was used as a substrate. The lipase activity can be determined by measuring the rate of increase in absorbance associated with the release of 4-nitrophenol by the action of the lipase. 20 mM 4-nitrophenyl octanoate in 20 mM Tris-HCl (pH: 7.0) was used as a substrate solution. The surfactant solution used was prepared by adding 0.1% (w/v) SDS or Triton X-100 to 20 mM Tris-HCl (pH: 7.0). 5 µL of the lipase solution adjusted to 2 ppm and 100 µL of the surfactant solution were mixed in each well of a 96-well assay plate, 10 µL of the substrate solution was added thereto, and the absorbance change (OD/min) at 405 nm was measured at 30°C. The difference ΔOD/min from the blank (enzyme-free sample) was used as the activity value. The activity value of each lipase in the surfactant solution was divided by the activity value when using 20 mM Tris-HCl (pH: 7.0) (activity in the buffer) in place of the surfactant solution, and then multiplied by 100. The resulting value was determined as the relative activity (%).

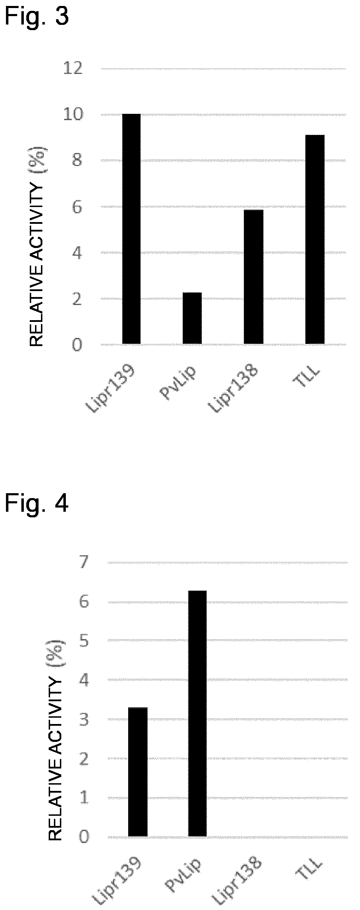

Figs. 3 and 4 show the results. The activity of TLL, and Lipr139, PvLip, and Lipr138, whose suitability for cleansing is disclosed in Patent Literatures 1, 2, and 3, was significantly inhibited in the presence of SDS. Similarly, their activity was also significantly inhibited in the presence of Triton X-100.

### (4) Measurement of activity in model cleansing liquid

20 mM 4-nitrophenyl octanoate in 20 mM Tris-HCl (pH: 7.0) was used as a substrate solution. The aqueous medium of Table 1 was used as a model cleansing liquid. 2 µL of the lipase solution adjusted to 4 ppm and 100 µL of a test liquid (model cleansing liquid or 20 mM Tris-HCl (pH: 7.0)) were mixed in each well of a 96-well assay plate, 10 µL of the substrate solution was added thereto, and the absorbance change (OD/min) at 405 nm was measured at 30°C. The difference ΔOD/min from the blank (enzyme-free sample) was determined. 10 µL of 20 mM Tris-HCl (pH: 7.0) containing from 31 to 500 µM of 4-nitrophenol was mixed with 100 µL of each test liquid, the absorbance at 405 nm was measured, and a calibration curve was created. From the calibration curve of each test liquid and the ΔOD/min value, the release rate (µM/min) of 4-nitrophenol per minute was calculated as the activity value. The activity value of each lipase when using the model cleansing liquid as the test liquid was divided by the activity value when using 20 mM Tris-HCl (pH: 7.0) as the test liquid (activity in the buffer), and then multiplied by 100. The resulting value was determined as the relative activity (%).

**[Table 1]**

| Component blended | mass% |
|---|---|
| Sulfosuccinic acid branched alkyl ester | 0.2 |
| Betaine | 2 |
| Butyl diglycol | 6.5 |
| Water | Balance |
| Total | 100 |

| | |
|---|---|
| - **Sulfosuccinic acid branched alkyl ester: sodium bis-(2-propylheptyl) sulfosuccinate** - **Betaine: lauryl(2-hydroxy-3-sulfopropyl)dimethyl betaine, Amphitol 20HD, manufactured by Kao Corporation** | |

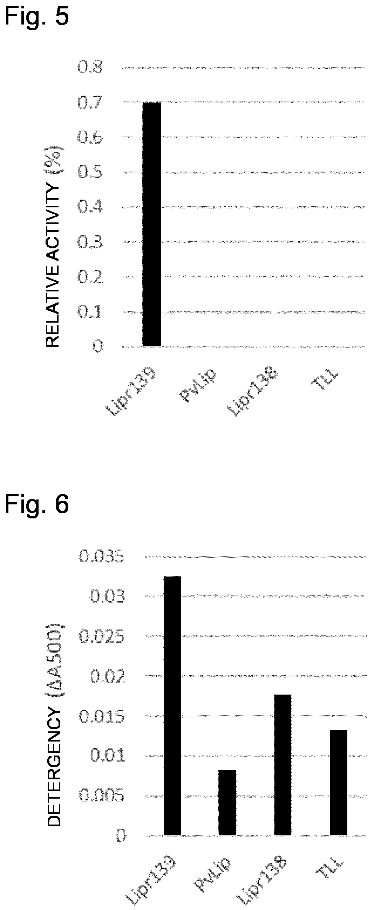

The results are shown in Fig. 5. The activity of TLL, PvLip, and Lipr138 was significantly inhibited in the model cleansing liquid, and their activity could not be confirmed. Although the activity of Lipr139 was detected, the activity was more inhibited than that in the presence of SDS and Triton X-100.

### (5) Cleansing evaluation in model cleansing liquid

Beef tallow (SIGMA, 03-0660) and rapeseed oil (SIGMA, 23-0450) were mixed at a weight ratio of 9:1, and dissolved in 3 times the amount of chloroform, followed by coloring with 0.2 wt% Sudan III, and the resultant was used as a model stain. 10 µL of the model stain was added dropwise to the bottom of each well of a 96-deep-well polypropylene plate, and chloroform was evaporated and dried to prepare a stain plate.

The cleansing liquid used was prepared by adding 1/100 volume of 240 ppm lipase solution to the model cleansing liquid shown in Table 1. 300 µL of the cleansing liquid was added slowly to each well of the stain plate, and allowed to stand at room temperature (about 22°C) for 15 minutes to perform immersion cleansing. 100 µL of the cleansing liquid was taken from each well so as not to be in contact with the stain on the bottom and transferred to a new 96-well plate. In order to quantify Sudan III in the model stain solubilized in the cleansing liquid by immersion cleansing, the absorbance at 500 nm (A500) was measured. A value ΔA500 obtained by subtracting A500 before cleansing from A500 after cleansing corresponds to the amount of oil released in the cleansing liquid, and can be used as an index for detergency.

Fig. 6 shows the detergency ΔA500 of each lipase. In (4) above, Lipr139, which had the highest activity in the model cleansing liquid, showed higher detergency in the model cleansing liquid in comparison with TLL, PvLip, and Lipr138.

### Example 1: Evaluation of existing lipases closely related to the Proteus/Yersinia clade and ancestral lipases

### (1) Design of ancestral lipases

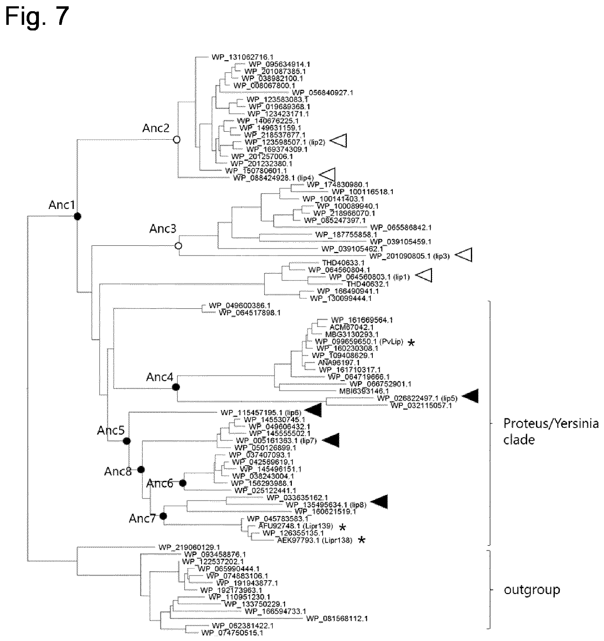

Phylogenetic analysis was carried out using a bacterial lipase sequence group homologous to Lipr139, PvLip, and Lipr138. Each lipase sequence of the bacterial lipase sequence group corresponds to each terminal node of the phylogenetic tree in Fig. 7. The default parameters of MAFFT v7.471 were used to construct multiple alignments, after which a conserved area was extracted with trimAl v1.4.rev15, and an evolutionary model was selected using ModelTest-NG v0.1.7. For the phylogenetic analysis, IQ-TREE multicore version 2.0.3 was used, and LG+I+G4 was specified for the evolutionary model. GRASP (https://github.com/bodenlab/GRASP) was used to estimate ancestral sequences. Fig. 7 shows a phylogenetic tree visualized using Figtree (http://tree.bio.ed.ac.uk/software/FigTree/).

Lipr139, PvLip, and Lipr138 (asterisks in Fig. 7) belonged to the *Proteus*/*Yersinia* clade including *Proteus* bacterium-derived and *Yersinia* bacterium-derived lipase sequences. From the phylogenetic tree, an existing sequence group belonging to the *Proteus*/*Yersinia* clade, i.e., Group 1 (Lip5, Lip6, Lip7, and Lip8; black arrowheads in Fig. 7), and an ancestral sequence group corresponding to their ancestors, i.e., Group 2 (Anc1, Anc4, Anc5, Anc6, Anc7, and Anc8; black circles in Fig. 7), as well as an existing sequence group not belonging to the *Proteus*/*Yersinia* clade, i.e., Group 3 (Lip1, Lip2, Lip3, and Lip4; white arrowheads in Fig. 7), and an ancestral sequence group corresponding to their ancestors, i.e., Group 4 (Anc2 and Anc3; white circles in Fig. 7), were randomly selected.

### (2) Construction of lipase expression plasmids

In the same manner as in Reference Example 1 (1), plasmids pHY-Lip1, pHY-Lip2, pHY-Lip3, pHY-Lip4, pHY-Lip5, pHY-Lip6, pHY-Lip7, pHY-Lip8, pHY-Anc1, pHY-Anc2, pHY-Anc3, pHY-Anc4, pHY-Anc5, pHY-Anc6, pHY-Anc7, and pHY-Anc8 were constructed from artificially synthesized lipase genes Lip1, Lip2, Lip3, Lip4, Lip5, Lip6, Lip7, Lip8, Anc1, Anc2, Anc3, Anc4, Anc5, Anc6, Anc7, and Anc8 (polynucleotides of respective SEQ ID NOs: 9, 11, 13, 15, 17, 19, 21, 23, 25, 27, 29, 31, 33, 35, 37, and 39, which respectively encode the amino acid sequences of SEQ ID NOs: 10, 12, 14, 16, 18, 20, 22, 24, 26, 28, 30, 32, 34, 36, 38, and 40), respectively.

### (3) Evaluation of each lipase

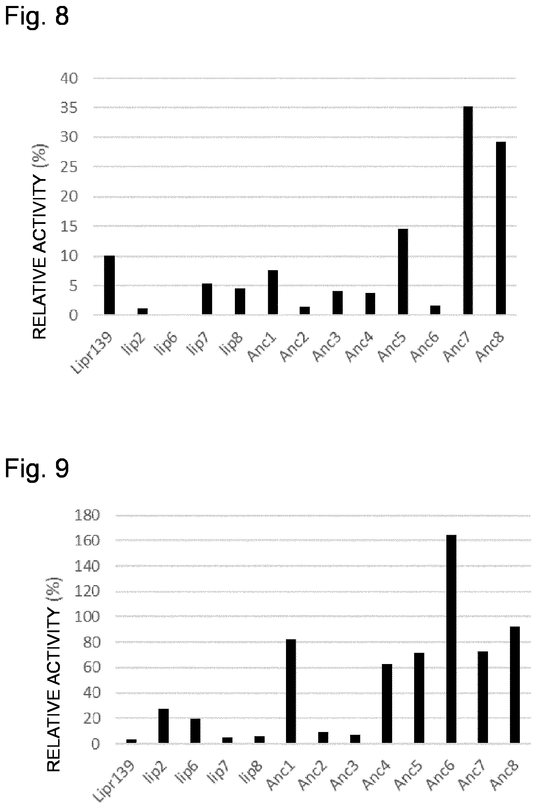

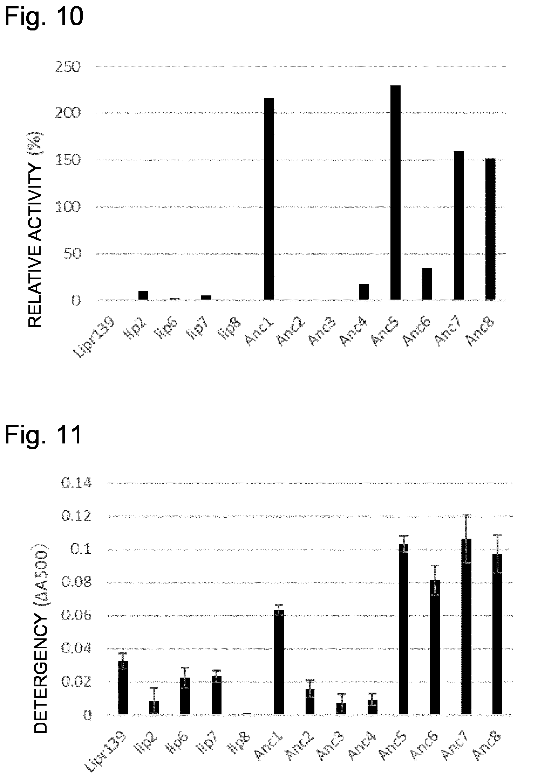

In the same manner as in Reference Example 1 (2), the lipase expression plasmids were each introduced into a *Bacillus subtilis* strain by the protoplast method, and the lipase-containing culture supernatant was collected and dialyzed. Regarding the sequence groups of Groups 1, 2, 3, and 4, the presence or absence of SDS-PAGE bands in the *Bacillus subtilis* supernatant was observed. As for the groups for which SDS-PAGE bands were observed, the relative activity in the surfactant (SDS or Triton X-100) solution, relative activity in the model cleansing liquid, and detergency in the model cleansing liquid were evaluated in the same manner as in Reference Example 1 (3), (4), and (5). The results are shown in Figs. 8 to 11 together with the results of Lipr139, and summarized in Table 2.

**[Table 2]**

| | Group1 | Group2 | Group3 | Group4 |
|---|---|---|---|---|
| Bands on SDS-PAGE confirmed | 3/4 | 6/6 | 1/4 | 2/2 |
| Relative activity in 0.1% SDS solution higher than Lipr139 | 0/3 | 3/6 | 0/1 | 0/2 |
| Relative activity in 0.1% Triton-X100 solution higher than Lipr139 | 3/3 | 6/6 | 1/1 | 2/2 |
| Relative activity in model cleansing liquid higher than Lipr139 | 2/3 | 6/6 | 1/1 | 1/2 |
| Detergency in model cleansing liquid higher than Lipr139 | 0/3 | 5/6 | 0/1 | 0/2 |
| Relative activity in 0.1% SDS solution of 7% or more | 0/3 | 4/6 | 0/1 | 0/2 |
| Relative activity in 0.1% Triton-X100 solution of 30% or more | 0/3 | 6/6 | 0/1 | 0/2 |
| Relative activity in model cleansing liquid of 30% or more | 0/3 | 5/6 | 0/1 | 0/2 |
| Detergency in model cleansing liquid of Δ0.05 or more | 0/3 | 5/6 | 0/1 | 0/2 |

Group 1, which was an existing sequence group belonging to the *Proteus*/*Yersinia* clade, showed a higher percentage of visible bands on SDS-PAGE, that is, a higher success rate of heterologous expression in *Bacillus subtilis,* in comparison with Group 3, which was also an existing sequence group. Sequences with successful heterologous expression included those whose relative activity in the Triton X-100 solution and model cleansing liquid was higher than that of Lipr139; however, none of them had higher relative activity in the SDS solution or a higher index for detergency ΔA500 in the model cleansing liquid than those of Lipr139. In addition, with reference to certain reference values, there was no sequence whose relative activity in the SDS solution, Triton X-100 solution, and model cleansing liquid exceeded 7%, 30%, and 30%, respectively. Further, there was no sequence whose index for detergency ΔA500 in the model cleansing liquid exceeded 0.05.

On the other hand, Group 2, which was an ancestral sequence group corresponding to the ancestors of the existing sequences belonging to the *Proteus*/*Yersinia* clade, showed a higher success rate of heterologous expression than that of Group 1, which was an existing sequence group belonging to the *Proteus*/*Yersinia* clade. In addition, sequences whose relative activity in the SDS solution, Triton X-100 solution, and model cleansing liquid, and whose index for detergency ΔA500 in the model cleansing liquid, exceeded those of Lipr139 could be obtained with a higher probability than Groups 1 and 3. With reference to certain reference values, sequences whose relative activity in the SDS solution, Triton X-100 solution, and model cleansing liquid exceeded 7%, 30%, and 30%, respectively, and whose index for detergency ΔA500 in the model cleansing liquid exceeded 0.05, could be obtained with a high probability of 4 or more out of 6 sequences. In Group 4, which was an ancestral sequence not corresponding to the ancestors of the existing sequences belonging to the *Proteus*/*Yersinia* clade, the success rate of heterologous expression was high; however, the probability of obtaining sequences whose relative activity in the SDS solution, Triton X-100 solution, and model cleansing liquid, and whose index for detergency ΔA500 in the model cleansing liquid, were higher than those of Lipr139 was equivalent to that of Groups 1 and 3, which were existing sequence groups. With reference to certain reference values, there was no sequence whose relative activity in the SDS solution, Triton X-100 solution, and model cleansing liquid exceeded 7%, 30%, and 30%, respectively, and whose index for detergency ΔA500 in the model cleansing liquid exceeded 0.05.

The above results indicated that by designing and evaluating sequences corresponding to the ancestors of existing sequences belonging to the *Proteus*/*Yersinia* clade, it is possible to obtain, with high efficiency, lipases having resistance to activity inhibition by a surfactant and showing an excellent cleansing effect even in the presence of a surfactant. Relative activity in various surfactant solutions is well correlated with detergency, and at least one of them can be evaluated to select useful lipases.

### Example 2: Evaluation of ancestral lipases belonging to the QGLP clade

### (1) Design of ancestral lipases

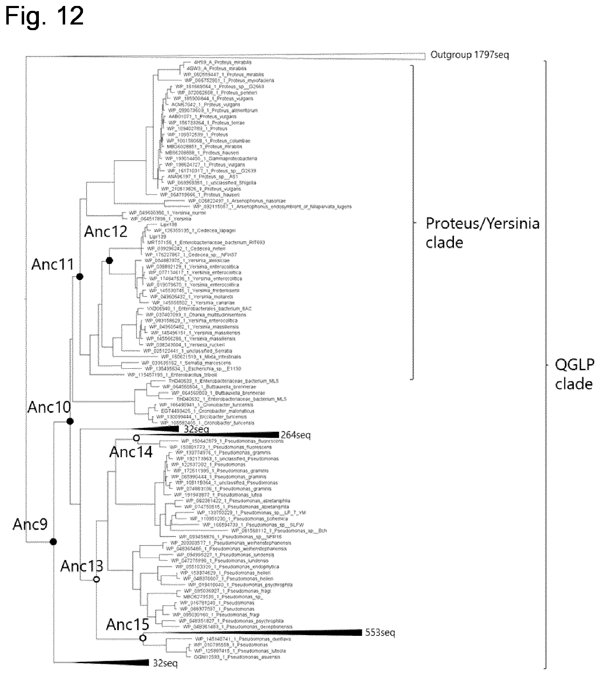

Phylogenetic analysis was carried out using a sequence group containing lipases more distantly related than those included in subgroups I.1 and I.2 of bacterial lipases. Each lipase sequence of the lipase sequence group corresponds to each terminal node of the phylogenetic tree in Fig. 12. The NCBI accession numbers of the lipase sequence group belonging to the QGLP clade in Fig. 12 are shown in Table 4 below. MAFFT-DASH (https://mafft.cbrc.jp/alignment/server/) was used to construct multiple alignments, after which a conserved area was extracted with trimAl v1.4.rev15, and an evolutionary model was selected using ModelTest-NG v0.1.7. For the phylogenetic analysis, IQ-TREE multicore version 2.0.3 was used, and LG+G4+F was specified for the evolutionary model. GRASP was used to estimate ancestral sequences. Fig. 12 shows a phylogenetic tree visualized using Figtree.

The *Proteus*/*Yersinia* clade was included in the QGLP clade. Of the QGLP clade, sequence groups more ancestral than Group 2 of Example 1 were evaluated. From the phylogenetic tree in Fig. 12, an ancestral sequence group belonging to the QGLP clade and corresponding to the ancestors of existing sequences belonging to the *Proteus*/*Yersinia* clade, i.e., Group 5 (Anc9, Anc10, Anc11, and Anc12; black circles in Fig. 12), and an ancestral sequence group belonging to the QGLP clade but not corresponding to the ancestors of existing sequences belonging to the *Proteus*/*Yersinia* clade, i.e., Group 6 (Ancl3, Ancl4, and Ancl5; white circles in Fig. 12), were randomly selected.

In the same manner as in Reference Example 1 (1), plasmids pHY-Anc9, pHY-Anc10, pHY-Anc11, pHY-Anc12, pHY-Ancl3, pHY-Ancl4, and pHY-Anc15 were constructed from artificially synthesized lipase genes Anc9, Anc10, Anc11, Anc12, Ancl3, Ancl4, and Anc15 (polynucleotides of respective SEQ ID NOs: 41, 43, 45, 47, 49, 51, and 53, which respectively encode the amino acid sequences of SEQ ID NOs: 42, 44, 46, 48, 50, 52, and 54), respectively.

### (2) Evaluation of each lipase

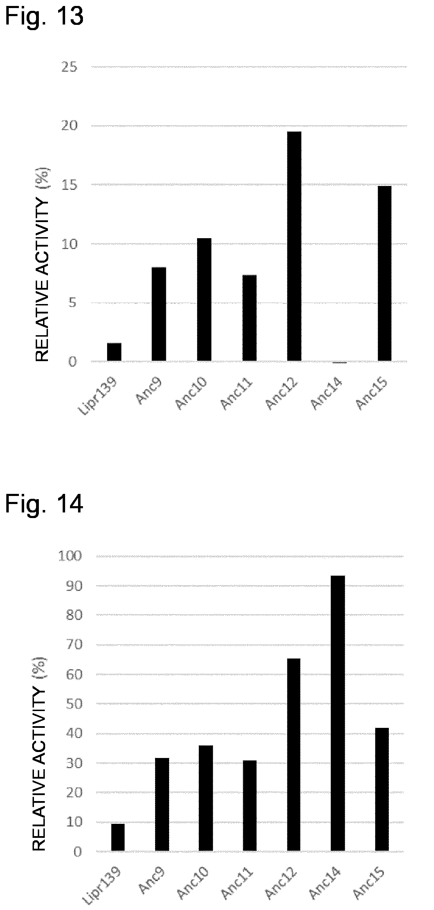

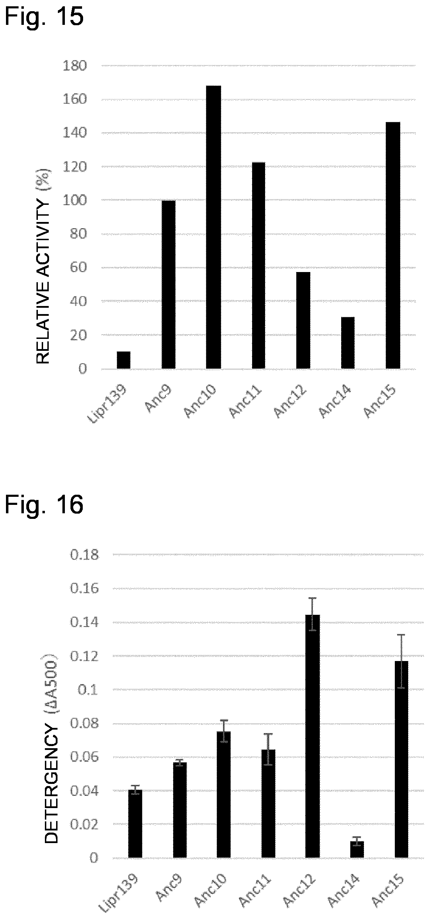

In the same manner as in Reference Example 1 (2), the lipase expression plasmids were each introduced into a *Bacillus subtilis* strain by the protoplast method, and the lipase-containing culture supernatant was collected and dialyzed. Regarding the sequence groups of Groups 5 and 6, the presence or absence of SDS-PAGE bands in the *Bacillus subtilis* supernatant was observed. As for the groups for which SDS-PAGE bands were observed, the relative activity in the surfactant (SDS or Triton X-100) solution, relative activity in the model cleansing liquid, and detergency in the model cleansing liquid were evaluated in the same manner as in Reference Example 1 (3), (4), and (5). The results are shown in Figs. 13 to 16 together with the results of Lipr139, and summarized in Table 3.

**[Table 3]**

| | Group5 | Group6 |
|---|---|---|
| Bands on SDS-PAGE confirmed | 4/4 | 2/3 |
| Relative activity in 0.1% SDS solution higher than Lipr139 | 4/4 | 1/2 |
| Relative activity in 0.1% Triton-X100 solution higher than Lipr139 | 4/4 | 2/2 |
| Relative activity in model cleansing liquid higher than Lipr139 | 4/4 | 2/2 |
| Detergency in model cleansing liquid higher than Lipr139 | 4/4 | 1/2 |
| Relative activity in 0.1% SDS solution of 7% or more | 4/4 | 1/2 |
| Relative activity in 0.1% Triton-X100 solution of 30% or more | 4/4 | 2/2 |
| Relative activity in model cleansing liquid of 30% or more | 4/4 | 2/2 |
| Detergency in model cleansing liquid of Δ0.05 or more | 4/4 | 1/2 |

Groups 5 and 6, which were ancestral sequence groups belonging to the QGLP clade, showed a higher success rate of heterologous expression than that of Groups 1 and 3, which were the existing sequence groups of Example 1. In addition, sequences whose relative activity in the SDS solution, Triton X-100 solution, and model cleansing liquid, and whose index for detergency ΔA500 in the model cleansing liquid, were higher than those of Lipr139 could be obtained with a higher probability than Groups 1 and 3. Similarly, with reference to certain reference values, sequences whose relative activity in the SDS solution, Triton X-100 solution, and model cleansing liquid exceeded 7%, 30%, and 30%, respectively, and whose index for detergency ΔA500 in the model cleansing liquid exceeded 0.05, could be obtained with a high probability. In particular, Group 5, which was an ancestral sequence group belonging to the QGLP clade and corresponding to the ancestors of existing sequences belonging to the *Proteus*/*Yersinia* clade, all of the evaluated sequences showed relative activity in the surfactant solution and detergency in the model cleansing liquid, both of which were higher than those of Lipr139 and also higher than certain reference values.

Therefore, it was indicated that in order to obtain, with high efficiency, lipases having resistance to activity inhibition by a surfactant and showing an excellent cleansing effect even in the presence of a surfactant, it is desirable to design and evaluate ancestral sequences belonging to the QGLP clade. In particular, it was indicated that it is desirable to design and evaluate ancestral sequences included in the QGLP clade and corresponding to the ancestors of existing sequences belonging to the *Proteus*/*Yersinia* clade.

### Reference Example 2: Identification of lipase sequence groups belonging to the QGLP clade and the Proteus/Yersinia clade using HMM profiles

### (1) Identification of lipase sequence groups belonging to the QGLP clade

The lipase sequence groups in Tabel 4 were compiled in fasta format to prepare a file QGLP.fasta. Using MAFFT v7.471, the command mafft --auto QGLP_mafft.fasta>QGLP.fasta was run to construct multiple alignments. Next, using HMMER version 3.3.2, the command hmmbuild --pnone --wnone QGLP_mafft.hmm QGLP_mafft.fasta was run to create the HMM profiles shown in Fig. 1, and further the command hmmpress QGLP_mafft.hmm was run for binarization. Next, ancestral sequences set forth in SEQ ID NOs: 26, 28, 30, 32, 34, 36, 38, 40, 42, 44, 46, 48, 50, 52, and 54 were compiled in fasta format to prepare a file Anc1-15.Fasta. The command hmmscan --tblout QGLP.tblout QGLP_mafft.hmm Anc1-15.Fasta was run to perform an ancestral sequence homology search for the HMM profiles, which define the QGLP clade. An example of the results is shown in Table 5. Ancl-15 all have an HMM score of 350 or more, indicating that they are ancestral sequences belonging to the QGLP clade. Whether any lipase sequences belong to the QGLP clade can be confirmed in this way.

**[Table 4-1]**

| | | | | | | | |
|---|---|---|---|---|---|---|---|
| 1 | WP_210813826.1 | 51 | WP_065925597.1 | 101 | WP_129437433.1 | 151 | WP_030131975.1 |
| 2 | WP_119428342.1 | 52 | WP_184745386.1 | 102 | WP_103304704.1 | 152 | WP_007903971.1 |
| 3 | WP_102354347.1 | 53 | WP_053134531.1 | 103 | WP_010487632.1 | 153 | WP_123358127.1 |
| 4 | WP_090367097.1 | 54 | WP_078823082.1 | 104 | WP_141129530.1 | 154 | WP_019650442.1 |
| 5 | WP_198760914.1 | 55 | WP_017526379.1 | 105 | WP_186685630.1 | 155 | WP_019582740.1 |
| 6 | WP_134660979.1 | 56 | WP_177038359.1 | 106 | WP_010444510.1 | 156 | WP_105343431.1 |
| 7 | MBN2993781.1 | 57 | WP_016972561.1 | 107 | WP_017904755.1 | 157 | WP_008014341.1 |
| 8 | WP_061435105.1 | 58 | WP_078740450.1 | 108 | WP_054058384.1 | 158 | WP_205889432.1 |
| 9 | WP_177051510.1 | 59 | WP_177011537.1 | 109 | WP_177083310.1 | 159 | WP_008067800.1 |
| 10 | WP_068935290.1 | 60 | WP_178918408.1 | 110 | WP_177064547.1 | 160 | WP_109513399.1 |
| 11 | WP_178952104.1 | 61 | WP_094949757.1 | 111 | WP_017130220.1 | 161 | WP_122471289.1 |
| 12 | WP_010213550.1 | 62 | WP_083355510.1 | 112 | WP_177098588.1 | 162 | WP_085730782.1 |
| 13 | WP_169377791.1 | 63 | WP_197625706.1 | 113 | WP_191489755.1 | 163 | WP_096794785.1 |
| 14 | WP_076954545.1 | 64 | WP_064450444.1 | 114 | WP_177090429.1 | 164 | WP_095944763.1 |
| 15 | WP_186605881.1 | 65 | RMO73767.1 | 115 | WP_040063897.1 | 165 | WP_137217663.1 |
| 16 | WP_099493486.1 | 66 | WP_179606149.1 | 116 | WP_121136227.1 | 166 | WP_108224420.1 |
| 17 | WP_025857324.1 | 67 | WP_145160225.1 | 117 | WP_044460920.1 | 167 | WP_150647810.1 |
| 18 | WP_071492965.1 | 68 | OAE10780.1 | 118 | WP_075117638.1 | 168 | SDS67067.1 |
| 19 | WP_058423538.1 | 69 | WP_213632955.1 | 119 | WP_078469586.1 | 169 | WP_196169223.1 |
| 20 | WP_122811438.1 | 70 | WP_047881394.1 | 120 | WP_020298721.1 | 170 | WP_196162589.1 |
| 21 | WP_065874322.1 | 71 | WP_181080432.1 | 121 | WP_123329641.1 | 171 | WP_070090451.1 |
| 22 | WP_207043581.1 | 72 | WP_034117481.1 | 122 | WP_103739542.1 | 172 | WP_070572768.1 |
| 23 | WP_124525588.1 | 73 | WP_098949712.1 | 123 | WP_108545971.1 | 173 | WP_043207969.1 |
| 24 | WP_135291402.1 | 74 | WP_186608287.1 | 124 | WP_110723596.1 | 174 | WP_196146969.1 |
| 25 | WP_122576365.1 | 75 | WP_116666799.1 | 125 | WP_123723431.1 | 175 | WP_084854260.1 |
| 26 | WP_192300230.1 | 76 | WP_150292245.1 | 126 | WP_159895161.1 | 176 | WP_209864976.1 |
| 27 | WP_122511318.1 | 77 | WP_177034491.1 | 127 | WP_212805893.1 | 177 | WP_119146246.1 |
| 28 | WP_122764184.1 | 78 | WP_099170148.1 | 128 | WP_161758778.1 | 178 | WP_186555571.1 |
| 29 | WP_159932698.1 | 79 | WP_057958178.1 | 129 | WP_025130514.1 | 179 | WP_108119364.1 |
| 30 | WP_193862042.1 | 80 | WP_170042492.1 | 130 | WP_068577037.1 | 180 | WP_074883106.1 |
| 31 | WP_048727328.1 | 81 | WP_038445622.1 | 131 | WP_085595454.1 | 181 | MBU1331390.1 |
| 32 | WP_194937363.1 | 82 | WP_198830441.1 | 132 | WP_185697545.1 | 182 | WP_069521524.1 |
| 33 | OYU06209.1 | 83 | WP_205480163.1 | 133 | KTC32633.1 | 183 | WP_149631159.1 |
| 34 | WP_185705154.1 | 84 | WP_154907267.1 | 134 | WP_095109572.1 | 184 | WP_203422000.1 |
| 35 | WP_105695204.1 | 85 | WP_065942865.1 | 135 | WP_150804166.1 | 185 | WP_150580960.1 |
| 36 | WP_056860497.1 | 86 | WP_169357973.1 | 136 | WP_150643708.1 | 186 | WP_181286216.1 |
| 37 | WP_058419511.1 | 87 | WP_214996843.1 | 137 | WP_123583267.1 | 187 | WP_030140553.1 |
| 38 | WP_099487941.1 | 88 | WP_155584921.1 | 138 | WP_145016406.1 | 188 | WP_007965094.1 |
| 39 | WP_170040318.1 | 89 | WP_010173943.1 | 139 | WP_019691815.1 | 189 | WP_038368405.1 |
| 40 | WP_200431992.1 | 90 | WP_084380903.1 | 140 | WP_123423738.1 | 190 | REF82643.1 |
| 41 | WP_138817078.1 | 91 | WP_149411120.1 | 141 | WP_103317021.1 | 191 | WP_122607776.1 |
| 42 | WP_138449246.1 | 92 | KAF1029505.1 | 142 | WP_071552086.1 | 192 | TDV41170.1 |
| 43 | WP_110623882.1 | 93 | KAF1011279.1 | 143 | WP_133337130.1 | 193 | WP_122599971.1 |
| 44 | WP_010563371.1 | 94 | WP_025808020.1 | 144 | WP_071174258.1 | 194 | WP_007912302.1 |
| 45 | WP_122718851.1 | 95 | WP_124318932.1 | 145 | WP_095055198.1 | 195 | WP_160060105.1 |
| 46 | WP_065900189.1 | 96 | WP_092301354.1 | 146 | WP_201020323.1 | 196 | WP_145255351.1 |
| 47 | WP_101265993.1 | 97 | WP_097139257.1 | 147 | WP_025262182.1 | 197 | MBU2157692.1 |
| 48 | WP_122673986.1 | 98 | WP_124349323.1 | 148 | WP_117165495.1 | 198 | MBP8236261.1 |
| 49 | WP_116627299.1 | 99 | WP_154848015.1 | 149 | WP_095634914.1 | 199 | PKM23476.1 |
| 50 | WP_090400833.1 | 100 | WP_154992764.1 | 150 | WP_123723247.1 | 200 | WP_090243828.1 |

**[Table 4-2]**

| | | | | | | | |
|---|---|---|---|---|---|---|---|
| 201 | WP_160015898.1 | 251 | WP_084942439.1 | 301 | WP_095129589.1 | 351 | WP_150767324.1 |
| 202 | WP_108488648.1 | 252 | WP_104447324.1 | 302 | WP_140894771.1 | 352 | WP_138967200.1 |
| 203 | MBA4288849.1 | 253 | WP_131062716.1 | 303 | WP_201256886.1 | 353 | WP_150744976.1 |
| 204 | MBU0807731.1 | 254 | WP_140676225.1 | 304 | WP_092269191.1 | 354 | HCS42733.1 |
| 205 | MBU0902221.1 | 255 | WP_123598507.1 | 305 | WP_133836297.1 | 355 | WP_083368972.1 |
| 206 | WP_123356750.1 | 256 | WP_169374309.1 | 306 | WP_149661221.1 | 356 | WP_127647698.1 |
| 207 | WP_052964328.1 | 257 | WP_133838415.1 | 307 | WP_169369231.1 | 357 | WP_025112509.1 |
| 208 | WP_008054018.1 | 258 | WP_150780601.1 | 308 | WP_054614993.1 | 358 | WP_102355372.1 |
| 209 | WP_103302469.1 | 259 | WP_088424928.1 | 309 | WP_163909863.1 | 359 | WP_047598562.1 |
| 210 | WP_201238646.1 | 260 | WP_033056967.1 | 310 | WP_213938076.1 | 360 | WP_123586499.1 |
| 211 | WP_201233598.1 | 261 | WP_056742766.1 | 311 | WP_123596504.1 | 361 | WP_201137478.1 |
| 212 | WP_123449124.1 | 262 | WP_192410995.1 | 312 | WP_123407746.1 | 362 | WP_095136143.1 |
| 213 | WP_150793451.1 | 263 | WP_134103651.1 | 313 | WP_095109907.1 | 363 | WP_098964173.1 |
| 214 | WP_016984193.1 | 264 | WP_201257006.1 | 314 | WP_016985400.1 | 364 | WP_202368339.1 |
| 215 | WP_187679953.1 | 265 | WP_201232380.1 | 315 | WP_039768776.1 | 365 | WP_041064542.1 |
| 216 | WP_150687183.1 | 266 | WP_123583083.1 | 316 | WP_192561219.1 | 366 | WP_145598458.1 |
| 217 | WP_042559378.1 | 267 | WP_103317644.1 | 317 | WP_064381365.1 | 367 | WP_198759281.1 |
| 218 | WP_210704967.1 | 268 | WP_019689368.1 | 318 | WP_011334467.1 | 368 | WP_047294775.1 |
| 219 | WP_210623923.1 | 269 | WP_123423171.1 | 319 | WP_083367158.1 | 369 | WP_108589644.1 |
| 220 | WP_150602442.1 | 270 | WP_150771722.1 | 320 | AAC15585.1 | 370 | WP_129394088.1 |
| 221 | WP_150615471.1 | 271 | WP_207283482.1 | 321 | WP_095667670.1 | 371 | WP_122660668.1 |
| 222 | WP_093106745.1 | 272 | WP_192343762.1 | 322 | NER60105.1 | 372 | WP_159812374.1 |
| 223 | WP_208937628.1 | 273 | WP_183679665.1 | 323 | WP_094995227.1 | 373 | WP_186745873.1 |
| 224 | WM67093.1 | 274 | WP_160621519.1 | 324 | WP_047275990.1 | 374 | WP_064118356.1 |
| 225 | WP_034154331.1 | 275 | WP_008015891.1 | 325 | WP_169430577.1 | 375 | WP_158835370.1 |
| 226 | REG09697.1 | 276 | WP_123722395.1 | 326 | WP_134827091.1 | 376 | WP_201204007.1 |
| 227 | WP_071172641.1 | 277 | WP_205887965.1 | 327 | WP_114883380.1 | 377 | WP_085695964.1 |
| 228 | ETF09651.1 | 278 | WP_003182775.1 | 328 | WP_085710056.1 | 378 | WP_115078094.1 |
| 229 | WP_122507447.1 | 279 | WP_109372539.1 | 329 | WP_065260517.1 | 379 | WP_207982878.1 |
| 230 | SIR12451.1 | 280 | WP_207965154.1 | 330 | WP_096821567.1 | 380 | NYH11622.1 |
| 231 | VVO70633.1 | 281 | WP_019578507.1 | 331 | WP_102622252.1 | 381 | WP_007952070.1 |
| 232 | VVO91342.1 | 282 | WP_105341132.1 | 332 | WP_192558126.1 | 382 | WP_085603303.1 |
| 233 | WP_101159607.1 | 283 | WP_090183483.1 | 333 | WP_085683857.1 | 383 | WP_102686085.1 |
| 234 | WP_133338193.1 | 284 | AHZ67197.1 | 334 | MBI3905764.1 | 384 | WP_158958940.1 |
| 235 | WP_186529252.1 | 285 | WP_131058719.1 | 335 | WP_116263029.1 | 385 | WP_122591156.1 |
| 236 | SDU40821.1 | 286 | WP_109632025.1 | 336 | WP_204933380.1 | 386 | WP_126362821.1 |
| 237 | WP_130909668.1 | 287 | WP_046027421.1 | 337 | WP_007990869.1 | 387 | WP_123531093.1 |
| 238 | WP_039758976.1 | 288 | WP_140671755.1 | 338 | WP_008001040.1 | 388 | WP_100848317.1 |
| 239 | WP_186546399.1 | 289 | WP_027923797.1 | 339 | WP_108230398.1 | 389 | WP_166226058.1 |
| 240 | WP_186647359.1 | 290 | WP_123431827.1 | 340 | WP_104451530.1 | 390 | PTT96111.1 |
| 241 | WP_122314061.1 | 291 | WP_007943528.1 | 341 | WP_007979674.1 | 391 | WP_103406622.1 |
| 242 | WP_033061677.1 | 292 | WP_076030789.1 | 342 | WP_028622713.1 | 392 | WP_196584950.1 |
| 243 | WP_099814366.1 | 293 | WP_074877516.1 | 343 | WP_150581814.1 | 393 | WP_150770563.1 |
| 244 | WP_160769180.1 | 294 | WP_102614318.1 | 344 | WP_149419151.1 | 394 | WP_160107046.1 |
| 245 | WP_136492322.1 | 295 | WP_111449801.1 | 345 | WP_059405910.1 | 395 | WP_175388554.1 |
| 246 | WP_122707764.1 | 296 | WP_008150916.1 | 346 | WP_157711207.1 | 396 | WP_095194715.1 |
| 247 | WP_123443925.1 | 297 | WP_134099871.1 | 347 | WP_201119742.1 | 397 | WP_207265668.1 |
| 248 | WP_166886851.1 | 298 | WP_129443710.1 | 348 | WP_154848404.1 | 398 | WP_137806336.1 |
| 249 | WP_094012226.1 | 299 | WP_046052987.1 | 349 | WP_154994892.1 | 399 | WP_110963231.1 |
| 250 | WP_214500929.1 | 300 | WP_150655666.1 | 350 | WP_150796479.1 | 400 | WP_175378129.1 |

**[Table 4-3]**

| | | | | | | | |
|---|---|---|---|---|---|---|---|
| 401 | WP_096511846.1 | 451 | WP_162436551.1 | 501 | WP_083375165.1 | 551 | WP_009394324.1 |
| 402 | WP_077045219.1 | 452 | WP_161669564.1 | 502 | WP_109627740.1 | 552 | WP_048365486.1 |
| 403 | WP_179057245.1 | 453 | WP_072062608.1 | 503 | WP_081568112.1 | 553 | WP_150755841.1 |
| 404 | EPA99094.1 | 454 | WP_185900844.1 | 504 | WP_092170251.1 | 554 | WP_056725379.1 |
| 405 | WP_188265515.1 | 455 | ACM67042.1 | 505 | WP_072405154.1 | 555 | WP_057712205.1 |
| 406 | WP_008063584.1 | 456 | WP_099073603.1 | 506 | WP_060738093.1 | 556 | WP_102669239.1 |
| 407 | WP_180205465.1 | 457 | AAB01071.1 | 507 | WP_092199292.1 | 557 | WP_007966968.1 |
| 408 | WP_077183256.1 | 458 | WP_156733364.1 | 508 | WP_192263187.1 | 558 | WP_166357800.1 |
| 409 | WP_034147148.1 | 459 | WP_109402789.1 | 509 | WP_122568416.1 | 559 | WP_166568648.1 |
| 410 | WP_150702177.1 | 460 | WP_100158068.1 | 510 | WP_017336297.1 | 560 | WP_055103320.1 |
| 411 | WP_075944719.1 | 461 | MBG6028851.1 | 511 | WP_176513265.1 | 561 | WP_153374629.1 |
| 412 | WP_212627422.1 | 462 | MBS6208888.1 | 512 | WP_007915924.1 | 562 | WP_048370007.1 |
| 413 | WP_130906873.1 | 463 | WP_193014450.1 | 513 | WP_062381422.1 | 563 | WP_019410040.1 |
| 414 | WP_093219467.1 | 464 | WP_198624727.1 | 514 | WP_074750515.1 | 564 | WP_095036927.1 |
| 415 | WP_111284029.1 | 465 | WP_161710317.1 | 515 | WP_133750229.1 | 565 | MBO6279535.1 |
| 416 | WP_085717744.1 | 466 | WP_139317771.1 | 516 | WP_110951230.1 | 566 | WP_016781240.1 |
| 417 | WP_057396963.1 | 467 | WP_143485908.1 | 517 | WP_166594733.1 | 567 | WP_088377597.1 |
| 418 | WP_150723650.1 | 468 | AEK97793.1 | 518 | WP_181130803.1 | 568 | WP_095030160.1 |
| 419 | WP_090457889.1 | 469 | WP_126355135.1 | 519 | WP_093464953.1 | 569 | WP_048351827.1 |
| 420 | WP_154946735.1 | 470 | WP_109752199.1 | 520 | WP_191942756.1 | 570 | WP_048361483.1 |
| 421 | WP_102671514.1 | 471 | WP_023378512.1 | 521 | WP_172610560.1 | 571 | MBO9549525.1 |
| 422 | WP_008051984.1 | 472 | WP_186686488.1 | 522 | HEF27657.1 | 572 | PVZ43294.1 |
| 423 | WP_095941924.1 | 473 | WP_170032973.1 | 523 | WP_065987765.1 | 573 | WP_103445864.1 |
| 424 | WP_046038589.1 | 474 | WP_186672608.1 | 524 | WP_120127758.1 | 574 | WP_085676867.1 |
| 425 | WP_056725093.1 | 475 | WP_196176065.1 | 525 | WP_081563906.1 | 575 | WP_152956967.1 |
| 426 | WP_116580591.1 | 476 | WP_114171224.1 | 526 | WP_198745892.1 | 576 | WP_060510596.1 |
| 427 | WP_126045605.1 | 477 | WP_129998162.1 | 527 | WP_110734950.1 | 577 | WP_133324590.1 |
| 428 | WP_095966784.1 | 478 | WP_011535914.1 | 528 | ANA96197.1 | 578 | WP_136916813.1 |
| 429 | PNB70467.1 | 479 | WP_166589498.1 | 529 | WP_069369381.1 | 579 | MBK4998622.1 |
| 430 | WP_007997918.1 | 480 | WP_110951072.1 | 530 | WP_201115105.1 | 580 | WP_197895263.1 |
| 431 | WP_108217577.1 | 481 | WP_203420539.1 | 531 | WP_214913561.1 | 581 | WP_079229890.1 |
| 432 | WP_150716861.1 | 482 | WP_092204822.1 | 532 | WP_130206328.1 | 582 | WP_186476399.1 |
| 433 | WP_160387931.1 | 483 | WP_095144777.1 | 533 | WP_095121881.1 | 583 | WP_167066917.1 |
| 434 | WP_085725823.1 | 484 | WP_095078188.1 | 534 | WP_163934885.1 | 584 | WP_090502341.1 |
| 435 | WP_103393994.1 | 485 | WP_103400928.1 | 535 | WP_065757910.1 | 585 | WP_012274427.1 |
| 436 | WP_110660363.1 | 486 | WP_093419067.1 | 536 | WP_077045350.1 | 586 | WP_172288689.1 |
| 437 | WP_007984659.1 | 487 | WP_182343284.1 | 537 | WP_095192091.1 | 587 | SUD73902.1 |
| 438 | WP_204933694.1 | 488 | WP_095169533.1 | 538 | WP_207261304.1 | 588 | WP_051101278.1 |
| 439 | WP_095058120.1 | 489 | WP_027617574.1 | 539 | WP_137807114.1 | 589 | WP_207828778.1 |
| 440 | WP_053160872.1 | 490 | WP_047600637.1 | 540 | WP_096513658.1 | 590 | WP_085625805.1 |
| 441 | WP_060540636.1 | 491 | WP_053155570.1 | 541 | WP_175387687.1 | 591 | APO84568.1 |
| 442 | WP_214382987.1 | 492 | WP_072397869.1 | 542 | PPA04198.1 | 592 | ESW37971.1 |
| 443 | WP_084320171.1 | 493 | WP_175363098.1 | 543 | WP_110966110.1 | 593 | SPO57723.1 |
| 444 | WP_017339135.1 | 494 | WP_208555211.1 | 544 | WP_175375490.1 | 594 | AUZ61430.1 |
| 445 | WM42796.1 | 495 | WP_041022499.1 | 545 | WP_074873313.1 | 595 | WP_110748059.1 |
| 446 | WP_150711872.1 | 496 | WP_144928475.1 | 546 | WP_145530745.1 | 596 | WP_086977219.1 |
| 447 | WP_008037106.1 | 497 | WP_047701010.1 | 547 | WP_049606432.1 | 597 | TCP75757.1 |
| 448 | WP_201188604.1 | 498 | WP_186610773.1 | 548 | WP_100123055.1 | 598 | WP_210667785.1 |
| 449 | WP_192308675.1 | 499 | WP_020293697.1 | 549 | WP_186730045.1 | 599 | WP_119371586.1 |
| 450 | WP_194692061.1 | 500 | WP_208670299.1 | 550 | WP_095056324.1 | 600 | WP_105946296.1 |

**[Table 4-4]**

| | | | | | | | |
|---|---|---|---|---|---|---|---|
| 601 | PYG80318.1 | 651 | WP_180204052.1 | 701 | WP_054044068.1 | 751 | WP_139642000.1 |
| 602 | WP_029380229.1 | 652 | WP_168757145.1 | 702 | WP_213940989.1 | 752 | RMM49078.1 |
| 603 | BBV99287.1 | 653 | WP_110753283.1 | 703 | WP_201193618.1 | 753 | WP_076407382.1 |
| 604 | WP_056794737.1 | 654 | WP_046819736.1 | 704 | WP_157714383.1 | 754 | WP_053155367.1 |
| 605 | WP_212623302.1 | 655 | WP_060542781.1 | 705 | WP_150712312.1 | 755 | WP_215000250.1 |
| 606 | SUD80440.1 | 656 | WP_140669675.1 | 706 | WP_192226945.1 | 756 | WP_100116518.1 |
| 607 | RIA40484.1 | 657 | WP_123435923.1 | 707 | WP_084319267.1 | 757 | WP_100141403.1 |
| 608 | WP_046787819.1 | 658 | WP_109785465.1 | 708 | WP_214380439.1 | 758 | WP_207851389.1 |
| 609 | WP_054900201.1 | 659 | WP_003200264.1 | 709 | WP_150654542.1 | 759 | WP_122599279.1 |
| 610 | WP_194790253.1 | 660 | WP_024616649.1 | 710 | WP_028621471.1 | 760 | WP_034151604.1 |
| 611 | BBH44085.1 | 661 | WP_214509111.1 | 711 | WP_123358648.1 | 761 | WP_064379133.1 |
| 612 | WP_016395214.1 | 662 | WP_063321973.1 | 712 | HCS42309.1 | 762 | WP_085703425.1 |
| 613 | WP_095063970.1 | 663 | WP_204126523.1 | 713 | WP_150763627.1 | 763 | WP_197869896.1 |
| 614 | EJN40259.1 | 664 | WP_175360076.1 | 714 | WP_150797066.1 | 764 | WP_085732695.1 |
| 615 | WP_027592559.1 | 665 | WP_203303577.1 | 715 | WP_150786046.1 | 765 | WP_119429421.1 |
| 616 | WP_125465734.1 | 666 | WP_150651737.1 | 716 | WP_150743959.1 | 766 | WP_096819784.1 |
| 617 | WP_129438912.1 | 667 | WP_084552574.1 | 717 | WP_139159437.1 | 767 | WP_007950616.1 |
| 618 | WP_075947778.1 | 668 | WP_007903453.1 | 718 | WP_077174617.1 | 768 | WP_065258816.1 |
| 619 | WP_212625908.1 | 669 | WP_007937075.1 | 719 | WP_174847536.1 | 769 | WP_135294854.1 |
| 620 | WP_056852417.1 | 670 | WP_205887604.1 | 720 | WP_131349996.1 | 770 | WP_204894408.1 |
| 621 | WP_150705410.1 | 671 | WP_105340440.1 | 721 | WP_071555126.1 | 771 | WP_128614213.1 |
| 622 | WP_053153810.1 | 672 | WP_111455289.1 | 722 | WP_054596820.1 | 772 | WP_201135926.1 |
| 623 | WP_126048959.1 | 673 | WP_030128295.1 | 723 | WP_123583865.1 | 773 | WP_103486494.1 |
| 624 | WP_102673140.1 | 674 | WP_038978744.1 | 724 | WP_145014688.1 | 774 | WP_192173963.1 |
| 625 | WP_160390487.1 | 675 | WP_042731334.1 | 725 | WP_123425990.1 | 775 | THD40633.1 |
| 626 | WP_150714575.1 | 676 | WP_139225388.1 | 726 | WP_093413981.1 | 776 | WP_064560804.1 |
| 627 | WP_008016841.1 | 677 | WP_131175865.1 | 727 | WP_027620328.1 | 777 | WP_064560803.1 |
| 628 | WP_111284338.1 | 678 | WP_131178734.1 | 728 | WP_095143320.1 | 778 | THD40632.1 |
| 629 | WP_057397772.1 | 679 | WP_083328615.1 | 729 | WP_103401594.1 | 779 | WP_166490941.1 |
| 630 | WP_085717455.1 | 680 | WP_082045834.1 | 730 | WP_095079600.1 | 780 | EGT4493425.1 |
| 631 | WP_103397147.1 | 681 | WP_090423679.1 | 731 | WP_159995222.1 | 781 | WP_130099444.1 |
| 632 | WP_085727339.1 | 682 | WP_010221637.1 | 732 | WP_092396111.1 | 782 | WP_105582405.1 |
| 633 | WP_108218455.1 | 683 | WP_123564972.1 | 733 | WP_214916934.1 | 783 | WP_053148009.1 |
| 634 | WP_093227091.1 | 684 | WP_038997417.1 | 734 | WP_123343200.1 | 784 | WP_072410190.1 |
| 635 | WP_034149668.1 | 685 | WP_130262897.1 | 735 | WP_025214520.1 | 785 | WP_122567271.1 |
| 636 | WP_110644916.1 | 686 | WP_045182439.1 | 736 | WP_092145655.1 | 786 | WP_053181534.1 |
| 637 | WP_075804720.1 | 687 | WP_192288788.1 | 737 | WP_186710430.1 | 787 | WP_053214722.1 |
| 638 | HCN46224.1 | 688 | WP_093504194.1 | 738 | WP_186729048.1 | 788 | WP_181287349.1 |
| 639 | WP_042120249.1 | 689 | WP_073262108.1 | 739 | WP_207961966.1 | 789 | WP_045155010.1 |
| 640 | WP_175654004.1 | 690 | WP_186642331.1 | 740 | WP_053183803.1 | 790 | WP_212797733.1 |
| 641 | WP_110971402.1 | 691 | WP_186609081.1 | 741 | WP_116832010.1 | 791 | WP_134922655.1 |
| 642 | WP_187755858.1 | 692 | WP_198724971.1 | 742 | WP_134923761.1 | 792 | AEV60646.1 |
| 643 | WP_129388408.1 | 693 | WP_201189444.1 | 743 | WP_053143937.1 | 793 | WP_214511421.1 |
| 644 | WP_008086200.1 | 694 | WP_201200186.1 | 744 | WP_215004881.1 | 794 | WP_150656603.1 |
| 645 | WP_201235727.1 | 695 | WP_123513986.1 | 745 | WP_210643926.1 | 795 | WP_150642879.1 |
| 646 | WP_166218123.1 | 696 | WP_123402390.1 | 746 | WP_135844741.1 | 796 | WP_150801773.1 |
| 647 | WP_125887415.1 | 697 | WP_149660614.1 | 747 | WP_047704151.1 | 797 | WP_158461071.1 |
| 648 | WP_033055591.1 | 698 | WP_133839394.1 | 748 | WP_144925797.1 | 798 | WP_095188295.1 |
| 649 | WP_007947556.1 | 699 | WP_169371846.1 | 749 | WP_041022599.1 | 799 | WP_187678561.1 |
| 650 | WP_008058918.1 | 700 | WP_163908054.1 | 750 | WP_175385976.1 | 800 | WP_158959352.1 |

**[Table 4-5]**

| | | | | | | | |
|---|---|---|---|---|---|---|---|
| 801 | WP_122593762.1 | 851 | WP_069144931.1 | 901 | WP_090731582.1 | 951 | GGM12593.1 |
| 802 | WP_127649048.1 | 852 | WP_122455030.1 | 902 | WP_122537202.1 | 952 | WP_039105459.1 |
| 803 | WP_025111810.1 | 853 | WP_122517461.1 | 903 | 4HS9_A | 953 | WP_110443908.1 |
| 804 | WP_200615046.1 | 854 | WP_122711216.1 | 904 | 4GW3_A | 954 | WP_100089940.1 |
| 805 | WP_201232147.1 | 855 | WP_099229543.1 | 905 | WP_060559447.1 | 955 | WP_174828609.1 |
| 806 | WP_212615178.1 | 856 | WP_024695539.1 | 906 | WP_066752901.1 | 956 | WP_085247397.1 |
| 807 | WP_024011393.1 | 857 | WP_073167920.1 | 907 | WP_064719666.1 | 957 | WP_065586842.1 |
| 808 | WP_093458876.1 | 858 | WP_083181044.1 | 908 | WP_026822497.1 | 958 | WP_201090805.1 |
| 809 | AFU92748.1 | 859 | WP_125830010.1 | 909 | WP_032115057.1 | | |
| 810 | WP_172611995.1 | 860 | WP_047574977.1 | 910 | WP_049600386.1 | | |
| 811 | WP_057448294.1 | 861 | WP_065833849.1 | 911 | WP_064517898.1 | | |
| 812 | WP_092147009.1 | 862 | WP_122663110.1 | 912 | MRT57156.1 | | |
| 813 | WP_116643272.1 | 863 | WP_087269766.1 | 913 | WP_039296242.1 | | |
| 814 | WP_123415102.1 | 864 | WP_122775365.1 | 914 | WP_176227867.1 | | |
| 815 | WP_123345072.1 | 865 | SQF94766.1 | 915 | WP_054887975.1 | | |
| 816 | WP_025211585.1 | 866 | WP_122725326.1 | 916 | WP_038892129.1 | | |
| 817 | WP_135843465.1 | 867 | WP_032631173.1 | 917 | WP_019079670.1 | | |
| 818 | WP_210642630.1 | 868 | WP_158158628.1 | 918 | WP_145555502.1 | | |
| 819 | WP_047227352.1 | 869 | WP_008368631.1 | 919 | VXD06940.1 | | |
| 820 | WP_122841591.1 | 870 | WP_136478375.1 | 920 | WP_037407093.1 | | |
| 821 | WP_194691557.1 | 871 | WP_203478832.1 | 921 | WP_083158629.1 | | |
| 822 | WP_095127360.1 | 872 | WP_038615149.1 | 922 | WP_049605482.1 | | |
| 823 | WP_140894944.1 | 873 | WP_107024180.1 | 923 | WP_145496151.1 | | |
| 824 | WP_134100200.1 | 874 | WP_128321517.1 | 924 | WP_145566286.1 | | |
| 825 | WP_111449230.1 | 875 | WP_028945710.1 | 925 | WP_038243004.1 | | |
| 826 | WP_149419295.1 | 876 | WP_133211624.1 | 926 | WP_025122441.1 | | |
| 827 | WP_046044796.1 | 877 | WP_116550692.1 | 927 | WP_033635162.1 | | |
| 828 | WP_059405771.1 | 878 | WP_138221182.1 | 928 | WP_135495634.1 | | |
| 829 | WP_085582807.1 | 879 | WP_063546355.1 | 929 | WP_115457195.1 | | |
| 830 | WP_123448429.1 | 880 | WP_153774107.1 | 930 | WP_133774976.1 | | |
| 831 | WP_053116825.1 | 881 | WP_186665961.1 | 931 | PZP20927.1 | | |
| 832 | WP_186651311.1 | 882 | WP_186732709.1 | 932 | WP_131340085.1 | | |
| 833 | WP_123419917.1 | 883 | WP_186660789.1 | 933 | WP_090619710.1 | | |
| 834 | WP_208938926.1 | 884 | WP_186713791.1 | 934 | WP_090935446.1 | | |
| 835 | WP_090280904.1 | 885 | WP_134690261.1 | 935 | WP_090900464.1 | | |
| 836 | WP_093105276.1 | 886 | WP_213658502.1 | 936 | WP_061290423.1 | | |
| 837 | WP_126359628.1 | 887 | WP_186536237.1 | 937 | WP_095646957.1 | | |
| 838 | WP_039756967.1 | 888 | RZI88609.1 | 938 | WP_095599771.1 | | |
| 839 | WP_076565471.1 | 889 | WP_181107316.1 | 939 | MBD7978701.1 | | |
| 840 | WP_041480702.1 | 890 | WP_103435873.1 | 940 | MBF0674628.1 | | |
| 841 | WP_101158354.1 | 891 | WP_104572987.1 | 941 | WP_027589898.1 | | |
| 842 | WP_186531652.1 | 892 | WP_087515825.1 | 942 | WP_017937086.1 | | |
| 843 | WP_090223942.1 | 893 | WP_090515544.1 | 943 | WP_187670377.1 | | |
| 844 | WP_108239476.1 | 894 | HBX54640.1 | 944 | WP_205348465.1 | | |
| 845 | WP_074751910.1 | 895 | WP_147171158.1 | 945 | WP_119891888.1 | | |
| 846 | WP_092410815.1 | 896 | WP_025164731.1 | 946 | WP_143489098.1 | | |
| 847 | WP_166595976.1 | 897 | WP_090448297.1 | 947 | WP_065990444.1 | | |
| 848 | WP_045486903.1 | 898 | WP_172151773.1 | 948 | WP_191943877.1 | | |
| 849 | WP_122702111.1 | 899 | WP_079204807.1 | 949 | WP_145140741.1 | | |
| 850 | WP_122473107.1 | 900 | \|WP_108104744.1 | 950 | WP_010795558.1 | | |

**[Table 5]**

| seq name | full seq score |
|---|---|
| Anc13 | 865.9 |
| Anc14 | 861.9 |
| Anc15 | 823.2 |
| Anc10 | 791.7 |
| Anc1 | 780.3 |
| Anc11 | 763.6 |
| Anc9 | 728.5 |
| Anc5 | 713.8 |
| Anc2 | 709.3 |
| Anc7 | 696.3 |
| Anc8 | 695.3 |
| Anc12 | 688.6 |
| Anc4 | 672.1 |
| Anc3 | 667.7 |
| Anc6 | 654.0 |

### (2) Identification of lipase sequence groups belonging to the Proteus/Yersinia clade

The lipase sequence groups in Table 6 were compiled in fasta format to prepare a file ProteusYersinia.fasta. Using MAFFT v7.471, the command mafft --localpair-maxiterate 16 ProteusYersinia.fasta>ProteusYersinia_mafft.fasta was run to construct multiple alignments. Next, using HMMER version 3.3.2, the command hmmbuild --pnone --wnone ProteusYersinia_mafft.hmm ProteusYersinia_mafft.fasta was run to create the HMM profiles shown in Fig. 2, and further the command hmmpress ProteusYersinia_mafft.hmm was run for binarization. Next, the lipase sequence groups shown in Fig. 12 were compiled in fasta format to prepare a file Lipase.fasta. The command hmmscan-tblout ProteusYersinia.tblout ProteusYersinia_mafft.hmm Lipase.Fasta was run to perform an existing sequence homology search for the HMM profiles, which define the *ProteusYersinia* clade. An example of the results is shown in Table 7. WP_210813826.1, Lipr139, and WP_026822497.1 all have an HMM score of 600 or more in full length, and belong to the *Proteus*/*Yersinia* clade. On the other hand, it can be said that WP_019650442.1 and WP_167404732.1, whose HMM scores are less than 600, do not belong to the *Proteus*/*Yersinia* clade. All of the existing sequences which are supposed to be in the *ProteusYersinia* clade in Figs. 7 and 12 have a score of 600 or more. Whether any lipase sequences belong to the Proteus/Yersinia clade can be confirmed in this way.

**[Table 6]**

| | | | | | | | |
|---|---|---|---|---|---|---|---|
| 1 | WP_198624727.1 | 16 | WP_049605482.1 | 31 | MRT57156.1 | 46 | MBS6208888.1 |
| 2 | WP_161710317.1 | 17 | WP_145496151.1 | 32 | WP_039296242.1 | 47 | WP_069369381.1 |
| 3 | WP_161669564.1 | 18 | WP_145566286.1 | 33 | WP_176227867.1 | 48 | WP_210813826.1 |
| 4 | WP_077174617.1 | 19 | WP_038243004.1 | 34 | WP_033635162.1 | 49 | WP_066752901.1 |
| 5 | WP_145555502.1 | 20 | WP_025122441.1 | 35 | WP_135495634.1 | 50 | WP_064719666.1 |
| 6 | WP_049600386.1 | 21 | WP_160621519.1 | 36 | WP_115457195.1 | 51 | WP_026822497.1 |
| 7 | WP_145530745.1 | 22 | WP_193014450.1 | 37 | WP_109402789.1 | 52 | 4HS9_A |
| 8 | WP_049606432.1 | 23 | WP_072062608.1 | 38 | ANA96197.1 | 53 | 4GW3_A |
| 9 | WP_054887975.1 | 24 | AAB01071.1 | 39 | WP_185900844.1 | | |
| 10 | WP_038892129.1 | 25 | WP_064517898.1 | 40 | ACM67042.1 | | |
| 11 | WP_174847536.1 | 26 | WP_032115057.1 | 41 | WP_099073603.1 | | |
| 12 | WP_019079670.1 | 27 | AEK97793.1 | 42 | WP_156733364.1 | | |
| 13 | VXD06940.1 | 28 | WP_126355135.1 | 43 | WP_109372539.1 | | |
| 14 | WP_037407093.1 | 29 | WP_060559447.1 | 44 | WP_100158068.1 | | |
| 15 | WP_083158629.1 | 30 | AFU92748.1 | 45 | MBG6028851.1 | | |

**[Table 7]**

| accession No. | full seq score |
|---|---|
| WP_210813826.1 | 997.8 |
| Lipr139 | 900.6 |
| WP_026822497.1 | 662.9 |
| WP_019650442.1 | 332.9 |
| WP_167404732.1 | 93.5 |

## Claims

1. A lipase search method comprising steps of:
designing lipase ancestral sequences by ancestral sequence reconstruction using a lipase sequence group;
measuring at least one of a lipase activity of lipases consisting of the designed ancestral sequences in the presence of a surfactant and a detergency thereof in the presence of a surfactant; and
comparing measured results with at least one of a lipase activity of a standard lipase in the presence of a surfactant and a detergency thereof in the presence of a surfactant.

2. The method according to claim 1, wherein the lipase sequence group is a bacterial lipase sequence group.

3. The method according to claim 1, wherein the lipase sequence group comprises at least one lipase sequence belonging to subfamily 1.1 or subfamily 1.2 of bacterial lipases.

4. The method according to claim 1, further comprising a step of selecting an ancestral sequence belonging to the QGLP clade from the designed ancestral sequences.

5. The method according to claim 4, wherein the ancestral sequence belonging to the QGLP clade corresponds to an ancestral sequence of a lipase existing sequence belonging to the *Proteus*/*Yersinia* clade.

6. The method according to claim 1, wherein the detergency is detergency at a low temperature.

7. The method according to claim 1, further comprising the steps of:
modifying the designed ancestral sequences to design modified ancestral sequences;
measuring at least one of a lipase activity of lipases consisting of the designed modified ancestral sequences in the presence of a surfactant and a detergency thereof in the presence of a surfactant; and
comparing measured results with at least one of a lipase activity of a standard lipase in the presence of a surfactant and a detergency thereof in the presence of a surfactant.

8. The method according to claim 7, wherein the modifying is amino acid substitution in the ancestral sequences or chimerization of the ancestral sequences.
